# EUROPEAN PATENT APPLICATION

(11) **EP 3 267 359 A1**
(43) Date of publication of application: **10.01.2018**
(21) Application number: 17179749.1
(22) Date of filing: 05.07.2017
(51) Int. Cl.: G06K 9/00, G06K 9/20, G06K 9/24

(54) **FINGERPRINT SENSOR, FINGERPRINT SENSING SYSTEM, AND SENSING SYSTEM USING LIGHT SOURCES OF DISPLAY PANEL**

(30) Priority: 06.07.2016 KR 20160085697; 05.08.2016 KR 20160100360; 30.12.2016 KR 20160184352
(71) Applicant: Samsung Electronics Co., Ltd., Gyeonggi-do 16677 (KR)
(72) Inventor: CHUNG, Dae-young, Seoul (KR); HWANG, Hee-chang, Seoul (KR); YOON, Kun-yong, Suwon-si, Gyeonggi-do (KR); KIM, Woon-bae, Seoul (KR); KIM, Bum-suk, Hwaseong-si, Gyeonggi-do (KR); JANG, Min, Hwaseong-si, Gyeonggi-do (KR); LEE, Min-chul, Seongnam-si, Gyeonggi-do (KR); KIM, Jung-woo, Osan-si, Gyeonggi-do (KR)
(74) Representative: Kuhnen & Wacker

(57) **Abstract**

At least some example embodiments provide a fingerprint sensor, a fingerprint sensor package, and a fingerprint sensing system using light sources of a display panel. The fingerprint sensor includes an image sensor including a plurality of sensor pixels, the sensor pixels configured to sense light reflected by a fingerprint and generate image information corresponding to the fingerprint and a pinhole mask defining a plurality of pinholes, wherein each of the pinholes forms a focus for transmitting the light reflected by the fingerprint to the image sensor, wherein light is emitted from a plurality of organic light-emitting diodes (OLEDs) and is reflected by the fingerprint.

## Description

### BACKGROUND

### 1. Field

Inventive concepts relate to a fingerprint sensor such as an optical fingerprint sensor and a sensor system, in particular a fingerprint sensor system or package using light sources of a display panel.

### 2. Description of the Related Art

A fingerprint sensor or a fingerprint recognition sensor is a sensor for sensing a fingerprint of a user. Recently, smart phones and wearable devices, on which a fingerprint sensor is mounted, have been widely used. Such a fingerprint sensor, which is mounted on the smart phone and the wearable device, may sense a fingerprint of the user using an electrical measurement method or an optical measurement method.

An optical fingerprint sensor using an optical measurement method may obtain a fingerprint image using a principle in which light reflected by ridges of a fingerprint and valleys between the ridges is sensed through an image sensor.

### SUMMARY

In order to apply such a fingerprint sensor to the smart phone and the wearable device, a size of the fingerprint sensor and improve the performance of fingerprint recognition is reduced.

Inventive concepts provide an on-display fingerprint sensor, a fingerprint sensor package, and a fingerprint sensing system including a fingerprint sensor.

According to an aspect of inventive concepts, there is provided a fingerprint sensor including an image sensor including a plurality of sensor pixels, the plurality of sensor pixels configured to sense light reflected by a fingerprint and generate image information corresponding to the fingerprint and a pinhole mask defining a plurality of pinholes, wherein each of the pinholes forms a focus for transmitting the light reflected by the fingerprint to the image sensor, and the light is emitted from a plurality of organic light-emitting diodes (OLEDs) and is reflected by the fingerprint.

According to another aspect of inventive concepts, there is provided a fingerprint sensor package, which is attached to one surface of a display panel including a plurality of organic light-emitting diodes (OLEDs) configured to generate light, the package including a package substrate, an image sensor on the package substrate, the image sensor defining a light-receiving region configured to sense the light generated by the OLEDs and reflected by a fingerprint, the light-receiving region configured to output an electrical signal, the image sensor further defining a logic region configured to generate image data by signal processing the electrical signal, generate a fingerprint image using the generated image data, and output the fingerprint image, and a pinhole mask between the plurality of OLEDs and the image sensor, the pinhole mask including a plurality of pinholes, the plurality of pinholes forming focuses for transmitting the light reflected by the fingerprint to the image sensor.

According to still another aspect of inventive concepts, there is provided a fingerprint sensing system including a display panel including light sources, the light sources configured to emit light having one or more colors in relation to a display operation, an image sensor attached to one surface of the display panel and configured to sense light from the light sources that is reflected by a fingerprint located on another surface of the display panel, the image sensor configured to generate image information according to a sensing result, and a pinhole mask between the display panel and the image sensor, the pinhole mask having a plurality of pinholes for forming a focus of the light reflected by the fingerprint, wherein, in a fingerprint sensing operation, the image sensor senses light having some of the one or more colors and generates the image information.

In at least one example embodiment, a sensing system includes a display panel configured to generate light and receive reflected light based on the generated light and a sensor, the sensor including a mask defining a plurality of holes, the mask configured to guide a first portion of the reflected light through the mask, a first side of the mask being on the display panel and an image sensor on a second side of the mask, the image sensor including a plurality of sensor pixels, the plurality of sensor pixels configured to sense the first portion of the reflected light and generate electrical signals based on the sensed light, the mask being configured to guide the first portion of the reflected light to the image sensor without a lens.

### BRIEF DESCRIPTION OF THE DRAWINGS

Example embodiments of inventive concepts will be more clearly understood from the following detailed description taken in conjunction with the accompanying drawings in which:
FIG. 1 is a structural diagram illustrating an example of a fingerprint sensing system including a fingerprint sensor according to an example embodiment of inventive concepts;
FIGS. 2A and 2B are diagrams illustrating a concept of a sensor pixel and an optical sensing region illustrated in FIG. 1 according to an example embodiment;
FIGS. 3A and 3B are diagrams illustrating examples of a light propagation path between a fingerprint pixel, a pinhole, and a sensor pixel according to an example embodiment;
FIG. 4 is a diagram illustrating an example in which a fingerprint sensor according to an example embodiment of inventive concepts is attached to a display panel;
FIGS. 5 and 6 are perspective views illustrating fingerprint sensing systems including a fingerprint sensor according to an example embodiment;
FIGS. 7 and 8 are diagrams illustrating example implementations of a pinhole mask according to an example embodiment of inventive concepts;
FIG. 9 is a diagram illustrating a structure of a fingerprint sensor package according to an example embodiment of inventive concepts;
FIGS. 10A and 10B are diagrams illustrating an example in which a fingerprint sensor package is attached to a display panel in a fingerprint sensing system according to an example embodiment;
FIGS. 11A and 11B are diagrams illustrating another example in which a fingerprint sensor package is attached to a display panel in a fingerprint sensing system according to an example embodiment;
FIG. 12 is a diagram illustrating still another example in which a fingerprint sensor package is attached to a display panel in a fingerprint sensing system according to an example embodiment;
FIGS. 13A and 13B are structural diagrams illustrating another example implementation of the fingerprint sensing system according to an example embodiment;
FIGS. 14 and 15 are diagrams illustrating example implementations of a fingerprint sensor in which a pinhole mask is embedded in an image sensor according to an example embodiment;
FIG. 16 is a diagram illustrating an example in which a fingerprint sensor package including a pinhole layer having an embedded structure is attached to a display panel according to an example embodiment;
FIGS. 17 to 24 are diagrams illustrating various structures of fingerprint sensor packages according to example embodiments of inventive concepts;
FIG. 25 is a diagram illustrating a fingerprint sensor according to an example embodiment of inventive concepts;
FIG. 26 is a diagram illustrating a structure of a fingerprint sensor package according to an example embodiment of inventive concepts;
FIG. 27 is a diagram illustrating a structure of a fingerprint sensor package according to another example embodiment of inventive concepts;
FIGS. 28A to 28C are diagrams illustrating a fingerprint sensing system including a fingerprint sensor package to which a micro-lens array is applied according to an example embodiment;
FIG. 29 is a diagram illustrating an example in which a fingerprint sensor package, to which a micro-lens array is applied, is mounted on a film according to an example embodiment;
FIGS. 30 to 32 are diagrams illustrating fingerprint sensing systems according to example embodiments of inventive concepts; and
FIG. 33 is a block diagram illustrating a processing system including the fingerprint sensors or fingerprint sensor packages according to example embodiments of inventive concepts.

### DETAILED DESCRIPTION OF EXAMPLE EMBODIMENTS

Hereinafter, some example embodiments of inventive concepts will be described in detail with reference to the accompanying drawings.

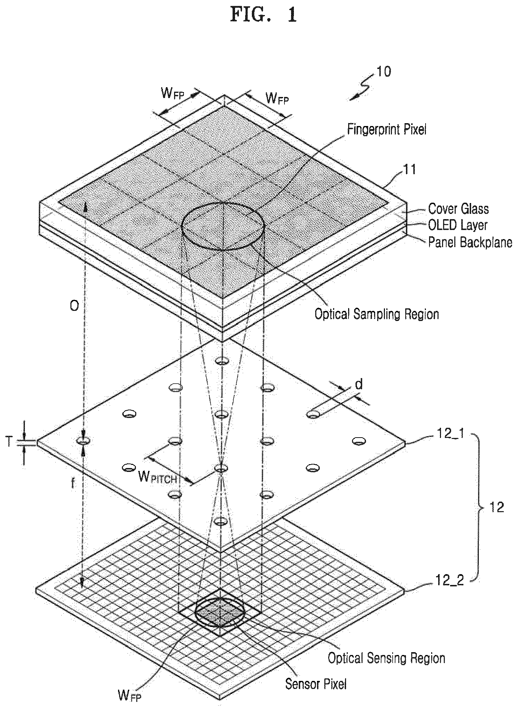

FIG. 1 is a structural diagram illustrating an example of a fingerprint sensing system including a fingerprint sensor according to an example embodiment of inventive concepts.

Referring to FIG. 1, a fingerprint sensing system 10 may include a display panel 11 and a fingerprint sensor 12. The fingerprint sensor 12 illustrated in FIG. 1 may be an optical fingerprint sensor which recognizes a fingerprint by sensing light, which is reflected by ridges of the fingerprint and valleys between the ridges, through an image sensor. According to an example embodiment, the fingerprint sensor 12 may include a pinhole mask 12_1 through which the light reflected by the fingerprint passes and an image sensor 12_2 which senses the light passing though the pinhole mask 12_1 to generate an electrical signal. According to an example embodiment, the pinhole mask 12_1 may be implemented as an opaque material so that light is allowed to pass through pinholes, whereas the light is blocked from passing through a region in which the pinholes are not formed. Also, according to an example embodiment, the pinhole mask 12_1 may be implemented as a material having a low reflectivity.

Various types of display panels may be applied to the display panel 11. According to an example embodiment, the display panel 11 may be an organic light-emitting diode (OLED) display panel including an OLED layer in which OLEDs which emit light having one or more colors and perform a display operation are formed. However, example embodiments of inventive concepts are not limited thereto, and the fingerprint sensing system 10 according to an example embodiment of inventive concepts may correspond to one of various types of display panels such as a liquid crystal display (LCD) panel which performs a display operation using general backlights or OLEDs. Alternatively, in addition to the above-described OLED display panel and LCD panel, when light emitted from a light source of a display panel is reflected by a fingerprint and is transmitted in a direction towards a backplane of the display panel (or in a direction towards a fingerprint sensor 12), the corresponding display panel may be applied to the display panel 11 according to an example embodiment of inventive concepts.

Meanwhile, the fingerprint sensor 12 may be implemented as a semiconductor chip or semiconductor package and attached to one surface of the display panel 11. According to one example embodiment, the image sensor 12_2 may be implemented as a semiconductor layer or semiconductor chip in which a plurality of photoelectric conversion elements (e.g., photodiodes (PDs), phototransistors, photo gates, pinned PDs, etc.) are formed. According to one example embodiment, the image sensor 12_2 may be a semiconductor layer in which an image sensor such as a complementary metal-oxide-semiconductor (CMOS) image sensor (CIS) and a charge coupled device (CCD) is implemented. In the following description, it is assumed that the photoelectric conversion elements in the image sensor 12_2 are implemented as PDs.

According to one example embodiment, in implementing the fingerprint sensor 12, in a process of packaging the image sensor 12_2, the pinhole mask 12_1 may be stacked on the image sensor 12_2. Alternatively, in a process of implementing the image sensor 12_2, the pinhole mask 12_1 may be stacked on one or more layers constituting the image sensor 12_2 in the form of a layer. That is, the fingerprint sensor 12 may be implemented in a form in which the pinhole mask 12_1 is embedded in the image sensor 12_2, and a packaging process may be performed on the image sensor 12_2 in which the pinhole mask 12_1 is embedded. That is, according to one example embodiment, the pinhole mask 12_1 and the image sensor 12_2 may be integrally formed.

The pinhole mask 12_1 may be implemented by various methods using a material having a low transmittance and a low reflectivity. For example, the pinhole mask 12_1 may be implemented using a material, that has a low reflectivity (or a high absorption rate) while blocking light and maintains its hardness even with temperature or humidity change. For example, the pinhole mask 12_1 may be implemented by forming pinholes after applying a titanium nitride (TiN) material on a silicon material. Alternatively, other non-silicon materials such as black nickel or anodized aluminium may be materials of the pinholes.

According to one example embodiment, the OLED layer included in the OLED display panel and the pinhole mask 12_1 may be substantially disposed in parallel. Accordingly, light emitted from the plurality of OLEDs in the OLED layer may be transmitted in a direction towards the fingerprint located on a cover glass, and the light reflected by the fingerprint may be transmitted to the pinhole mask 12_1 within an angle of view formed by the pinhole in the pinhole mask 12_1. Accordingly, in fingerprint sensors according to example embodiments of inventive concepts, it is not necessary to provide a separate light guiding unit for controlling a path through which light is transmitted for fingerprint sensing.

The fingerprint sensor 12 senses a fingerprint which comes in contact with the display panel 11 or is located near the display panel 11. In the fingerprint sensing system 10 according to example embodiments of inventive concepts, a fingerprint touched on a display of a wearable device such as a smart phone or the like may be recognized without the need of mounting a separate button for fingerprint recognition. For example, when the display panel 11 corresponds to an OLED display panel and the fingerprint of a user is placed on the cover glass of the display panel 11, the light emitted from the OLED in the display panel 11 may be transmitted to and reflected by the fingerprint of the user as a light source, and the reflected light may be transmitted through the backplane of the display panel and transmitted to the image sensor 12_2 through the pinhole mask 12_1.

The image sensor 12_2 includes a plurality of sensor pixels, and each of the sensor pixels senses lights reflected by different regions of the fingerprint, and generates electrical signals corresponding to the sensed lights. Each of the sensor pixels may generate an electrical signal corresponding to light reflected by ridges of the fingerprint, or generate an electrical signal corresponding to light reflected by valleys between the ridges. An amount of light sensed in the PD may vary according to a shape of the fingerprint by which the light is reflected, and electrical signals having different levels may be generated according to the amount of sensed light. That is, the electrical signals generated from the plurality of sensor pixels may each include contrast information (or image information), it may be determined whether a region corresponding to each of the sensor pixels is a ridge or a valley through a processing operation on the electrical signal, and the entire fingerprint image may be configured by combining the determined information.

Regions of the fingerprint which are optically sampled in the fingerprint sensing system 10 may be defined. For example, a plurality of fingerprint pixels W_{FP} may be defined to correspond to the plurality of sensor pixels of the image sensor 12_2, and each of the fingerprint pixels W_{FP} may correspond to an object region shown by one pinhole and one sensor pixel. A shape and size of the fingerprint pixel W_{FP} corresponding to each pinhole may be determined according to variable factors such as a distance between the display panel 11 and the pinhole mask 12_1, a distance between the pinhole mask 12_1 and the image sensor 12_2, a thickness of the pinhole mask 12_1, a diameter of the pinhole, a shape of the pinhole, and the like.

Each of the fingerprint pixels W_{FP} may correspond to one pinhole in the pinhole mask 12_1. A region which reflects light that may pass through one pinhole may be included in each of the fingerprint pixels W_{FP}, and the corresponding region may be defined as an optical sampling region. According to the optical sampling region, an optical sensing region corresponding to the optical sampling region may also be defined in the image sensor 12_2. For example, the optical sensing region may include the sensor pixel.

Meanwhile, although the fingerprint pixels W_{FP} are illustrated as being located in the entire region of the display panel 11 in FIG. 1, example embodiments of inventive concepts are not limited thereto. For example, the fingerprint pixels W_{FP} may be located only in some regions of the display panel 11, and thus the fingerprint may be sensed when the fingerprint of the user is located in a specific region of the display panel 11.

Also, each of the plurality of pinholes in the pinhole mask 12_1 may correspond to each of the plurality of sensor pixels in the image sensor 12_2. For example, one sensor pixel corresponding to one pinhole may include one PD. Alternatively, one sensor pixel corresponding to one pinhole may include two or more PDs. In FIG. 1, an example in which one sensor pixel includes a plurality of PDs and the sensor pixel in the optical sensing region includes a plurality of PDs is illustrated. That is, the plurality of pinholes of the pinhole mask 12_1 may be formed to be mapped to the plurality of pixels of the image sensor 12_2, light reflected by the fingerprint pixel in the optical sampling region may be sensed by one or more PDs in the sensor pixel, and the entire image of the fingerprint may be reconfigured by processing the electrical signals generated from the plurality of sensor pixels.

According to one example embodiment, a region in the image sensor 12_2 may be defined to correspond to each of the fingerprint pixels W_{FP}, and the region corresponding to each of the fingerprint pixels W_{FP} may include a plurality of PDs. Also, the sensor pixel may correspond to a region including at least some of the plurality of PDs corresponding to the fingerprint pixels W_{FP}. That is, one sensor pixel senses light corresponding to the fingerprint pixel W_{FP} corresponding thereto, and light corresponding to another fingerprint pixel W_{FP} is prevented from overlapping. In FIG. 1, an example in which a region corresponding to each of the fingerprint pixels W_{FP} includes 5*5 of PDs and the sensor pixel includes 3*3 of PDs as parts thereof is illustrated, and a fingerprint image (or a partial fingerprint image) may be configured based on electrical signals from the 3*3 of PDs with respect to the region corresponding to each of the fingerprint pixels W_{FP}.

Meanwhile, the fingerprint sensing system 10 is described as sensing the fingerprint of the user. However, example embodiments of inventive concepts are not limited thereto. For example, when a predetermined and/or desired object is located on the display panel 11, the fingerprint sensor 12 may sense light reflected by the predetermined and/or desired object to generate a sensing result thereof. When each fingerprint pixel of the fingerprint sensor 12 generates image data as a sensing result, an image of the object located on the display panel 11 may be reconfigured using the image data generated from each fingerprint pixel of the fingerprint sensor 12.

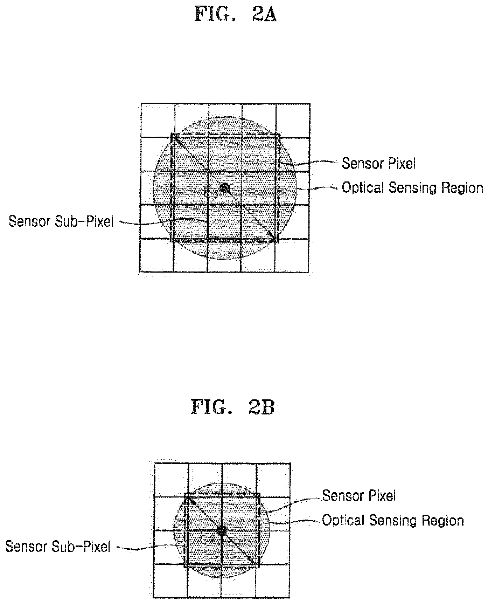

FIGS. 2A and 2B are diagrams illustrating a concept of the sensor pixels and the optical sensing region illustrated in FIG. 1. In FIGS. 2A and 2B, the sensor pixels corresponding to one fingerprint pixel are illustrated, and it is assumed that each of the sensor pixels includes a plurality of PDs.

An example in which one sensor pixel includes 3*3 of PDs is illustrated in FIG. 2A, and an example in which one sensor pixel includes 2*2 of PDs is illustrated in FIG. 2B. For example, when it is assumed that a plurality of PDs constitute one sensor pixel, a region corresponding to each PD may be referred to a sensor sub-pixel.

According to an example embodiment of inventive concepts, a center of the sensor pixel or a center of the optical sensing region may be aligned with a center of the pinhole. Also, the optical sensing region to which the reflected light in the optical sampling region of the fingerprint pixel is transmitted may be located over the plurality of PDs. For example, a diameter of the optical sensing region may have a value corresponding to the diameter F_{d}. The diameter F_{d} of the optical sensing region may be adjusted through various parameters such as a distance between the display panel and the pinhole mask, a distance between the pinhole mask and the image sensor, a shape of the pinhole, and the like. As described above, the diameter F_{d} of the optical sensing region is determined so that light from two or more fingerprint pixels does not overlap each sensor pixel.

The number of PDs in one sensor pixel in which light is actually sensed may be determined according to the diameter F_{d} of the optical sensing region. An electrical signal corresponding to one fingerprint pixel may be generated according to sensing results from the plurality of PDs. According to one example embodiment, the electrical signal may be generated through a process of merging data values of the plurality of PDs included in one sensor pixel.

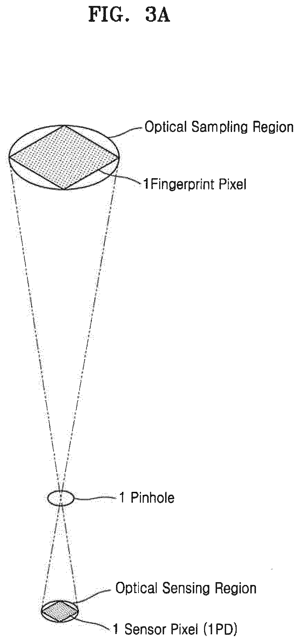

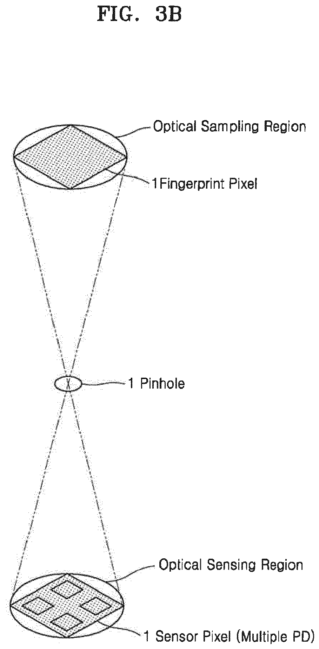

FIGS. 3A and 3B are diagrams illustrating examples of a light propagation path between a fingerprint pixel (or an optical sampling region), a pinhole, and a sensor pixel (or an optical sensing region). FIG. 3A illustrates an example in which one sensor pixel includes one PD, and FIG. 3B illustrates an example in which one sensor pixel includes a plurality of PDs.

Referring to FIGS. 3A and 3B, a pinhole may be located between the fingerprint pixel on the display panel and the sensor pixel on the image sensor, and the pinhole may form a focus of light transmitted between the fingerprint pixel and the sensor pixel. Light emitted from a light source is reflected by a region of the fingerprint (e.g., the optical sampling region) located in the fingerprint pixel and passes through the pinhole, and the light passing through the pinhole is provided to the sensor pixel corresponding to the fingerprint pixel. The PD included in the sensor pixel senses the light passing through the pinhole, and generates an electrical signal corresponding to the sensed light. In FIG. 3A, since one PD is formed in one sensor pixel, contrast data corresponding to the fingerprint pixel may be generated through a processing operation on the electrical signal generated from the one PD.

When it is assumed that a size of the PD is fixed, the number of PDs included in one sensor pixel may vary according to the arrangement of the fingerprint pixel, pinhole, and sensor pixel. For example, when the pinhole is located relatively close to the sensor pixel, the size of the sensor pixel corresponding to one fingerprint pixel may be reduced, and in some cases, one PD may be disposed in one sensor pixel. On the other hand, as illustrated in FIG. 3B, when a distance between the pinhole and the sensor pixel is relatively large, the size of the sensor pixel may be increased, and thus a plurality of PDs may be disposed in one sensor pixel. For example, an example in which four PDs are included in one sensor pixel is illustrated in FIG. 3B. One sensor pixel may generate an electrical signal including image information corresponding to one fingerprint pixel, and generate, for example, an electrical signal corresponding to one fingerprint pixel by merging respective sensing results by the four PDs. Contrast or image data corresponding to each fingerprint pixel may be generated through a processing operation on the electrical signal generated in this way.

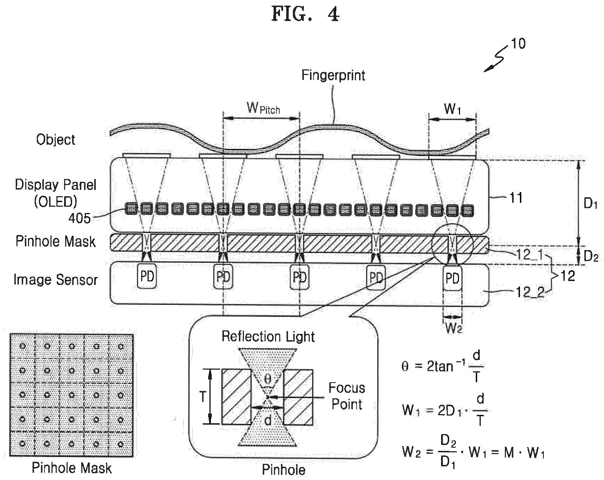

FIG. 4 is a diagram illustrating an example in which a fingerprint sensor according to an example embodiment of inventive concepts is attached to a display panel. As described above, the fingerprint sensor 12 may include the pinhole mask 12_1 and the image sensor 12_2, and may correspond to a semiconductor device when the pinhole mask 12_1 and the image sensor 12_2 are manufactured by a semiconductor process. Also, in a form of a fingerprint illustrated in FIG. 4, a portion which comes in contact with the display panel 11 may correspond to a ridge of the fingerprint, and a portion which does not come in contact with the display panel 11 may correspond to a valley of the fingerprint.

In some of the following example embodiments, a predetermined and/or desired interval between fingerprint pixels is illustrated for convenience of description of the light propagation path, but example embodiments of inventive concepts are not limited thereto. For example, boundaries of the fingerprint pixels may be defined to come in contact with each other so that the entire image of the fingerprint may be configured as illustrated in FIG. 1.

Referring to FIG. 4, the fingerprint sensor 12 may be attached to the backplane of the display panel 11, and may be, for example, a semiconductor layer in which the image sensor 12_2 such as a CIS is implemented. A plurality of sensor pixels each including a plurality of PDs may be formed in the image sensor 12_2, and, for example, the PDs may be formed in a semiconductor substrate (Sub) through an ion implantation process. In FIG. 4, an example of the optical sampling region corresponding to one fingerprint pixel and the optical sensing region corresponding to a sensor pixel region including one PD is illustrated.

Meanwhile, the pinhole mask 12_1 may be located between the display panel 11 and the image sensor 12_2. The pinhole mask 12_1 may be referred to as a pinhole mask layer since the pinhole mask 12_1 is implemented as a separate layer. In the example of FIG. 4, although the pinhole mask 12_1 in which pinholes having a 5*5 of structure are formed is illustrated for convenience of illustration, a substantially larger number of pinholes may be formed in the pinhole mask 12_1 in an array structure. For example, the pinhole mask 12_1 including approximately 200*200 of pinholes in an array structure may be used in consideration of the size of the fingerprint.

According to one example embodiment, the pinhole mask 12_1 may be stacked on the image sensor 12_2 in a process of implementing the image sensor 12_2. In this case, an intermediate layer such as a transparent passivation layer through which light may be transmitted and an oxide layer may be interposed between the pinhole mask 12_1 and the image sensor 12_2. That is, when the pinhole mask 12_1 is implemented to be embedded in the image sensor 12_2, the image sensor 12_2 may be described as including the pinhole mask 12_1. Also, a distance between the pinhole and the sensor pixel may be defined based on a thickness of the intermediate layer in the embedded structure described above, and a size of the sensor pixel corresponding to one fingerprint pixel may be defined based on the distance.

The display panel 11 may correspond to one of various types of display panels which may generate light toward the fingerprint and transmit the light reflected by the fingerprint in a direction towards the backplane of the display panel 11 (or in a direction towards the fingerprint sensor). According to one example embodiment, the display panel 11 may be an OLED panel, and include a plurality of OLEDs 405 as light sources. For example, the plurality of OLEDs 405 may be light emitting elements that emit light in various colors by themselves, and the plurality of OLEDs that emit red color (R), green color (G), and blue color (B) may be included in the display panel 11. At least one of the plurality of OLEDs 405 included in the display panel 11 may be used as light sources for sensing the fingerprint.

Although not illustrated in FIG. 4, when RGB OLEDs are all used for sensing the fingerprint, color filters (not illustrated) may be further implemented in the image sensor 12_2 so that white light is generated from the display panel 11 and each of the PDs senses light corresponding to a specific color. For example, red, green, and blue filters may be implemented to correspond to the plurality of PDs.

According to one example embodiment, the plurality of pinholes formed in the pinhole mask 12_1 are aligned with the plurality of sensor pixels of the image sensor 12_2. Also, a diameter W₁ of the optical sampling region including a size of the fingerprint pixel and a diameter W₂ of the optical sensing region including a size of the sensor pixel may be determined by a distance between the pinhole mask 12_1 and the fingerprint, that is, a pinhole-to-fingerprint distance D1, a distance between the pinhole mask 12_1 and the image sensor 12_2, that is, a pinhole-to-sensor distance D2, a diameter of the pinhole, and a thickness of the pinhole mask. Also, a pitch W_{pitch} between the fingerprint pixels may also be considered when the diameter W₁ of the optical sampling region is determined. According to an example embodiment of inventive concepts, the pinhole mask 12_1 including the plurality of pinholes may be disposed on the image sensor 12_2 in order to form a focus instead of applying a lens as a focus forming unit in the image sensor 12_2.

Specifically, when the fingerprint of the user is located on the display panel 11, light emitted from the OLEDs 405 in the display panel 11 is emitted in a direction towards the cover glass of the display panel 11, the emitted light is reflected by ridges and valleys of the fingerprint located on the cover glass and transmitted toward the backplane of the display panel 11, and the image sensor 12_2 senses the light transmitted through the backplane of the display panel 11 and the pinholes. Accordingly, an image of the fingerprint may be captured by the image sensor 12_2. According to example embodiments of inventive concepts, the fingerprint sensor 12 may be implemented as an ultra-thin optical sensor, and thus the image of the fingerprint may be captured by the fingerprint sensor 12 without a focusing lens.

Meanwhile, in aligning the fingerprint pixels, pinholes, and sensor pixels, a diameter d and thickness T of the pinhole correspond to parameters which determine an angle of view θ with respect to an upper surface and a lower surface based on the pinhole. Also, in addition to the determined angle of view θ, the above-described distance D1 between the pinhole mask 12_1 and the fingerprint, and the above-described distance D2 between the pinhole mask 12_1 and the image sensor 12_2 may correspond to parameters which determine the diameter W₁ of the optical sampling region including the size of the fingerprint pixel and the diameter W₂ of the optical sensing region including the size of the sensor pixel. Also, the angle of view θ, the diameter W₁ of the optical sampling region corresponding to the size of the fingerprint pixel, and the diameter W₂ of the optical sensing region corresponding to the size of the sensor pixel may be calculated using formulas illustrated in FIG. 4, where M denotes a magnification and may be defined as D2/D1.

The fingerprint sensor and the fingerprint sensing system illustrated in the above-described example embodiments may be applied to various fields. As an application of inventive concepts, the fingerprint sensor and the fingerprint sensing system may be applied to a system including a display panel, and, for example, may be applied to a system in which light emitted from a light source included in a display panel has a transmittance in a backplane direction of the display panel. Also, as an example of the application, at least some example embodiments of inventive concepts may be applied to a smart phone (including a tablet computer) or a wearable device (e.g., a smart watch). Alternatively, the fingerprint sensor and the fingerprint sensor package according to example embodiments of inventive concepts may be applied to an Internet of things (IoT) type ultra-thin camera.

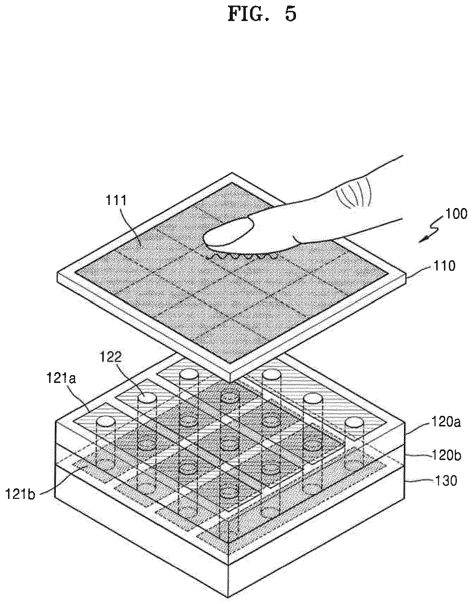

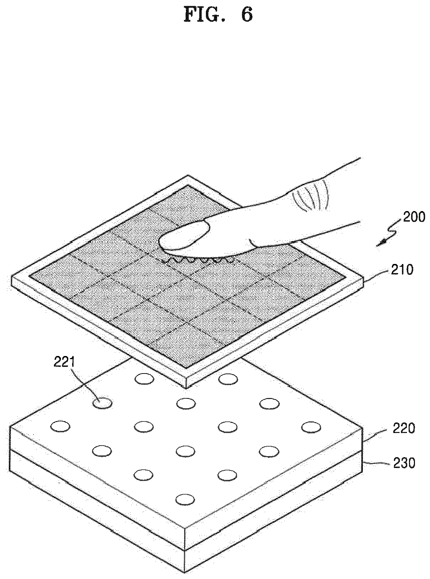

FIGS. 5 and 6 are perspective views illustrating fingerprint sensing systems including a fingerprint sensor according to an example implementation.

Referring to FIG. 5, a fingerprint sensing system 100 may include a display panel 110 and a fingerprint sensor. The display panel 110 may include a plurality of pixels 111 for implementing an image, and the pixels 111 may each include the light source in the above-described embodiment. For example, the light source included in each of the pixels 111 may be an OLED for expressing a predetermined and/or desired color.

Meanwhile, the fingerprint sensor may be implemented using a semiconductor wafer. As illustrated in FIG. 5, the fingerprint sensor may include one or more layers (e.g., first and second layers 120a and 120b) in which metal wirings for transmitting various signals are formed and a device layer 130 in which PDs are formed. For example, the first and second layers 120a and 120b may include metal wirings 121a and 121b implemented with a material through which light dose not pass, respectively, and the metal wirings 121a and 121b may be used for blocking the light reflected and transmitted by the fingerprint. Meanwhile, a plurality of pinholes 122 for forming a focus of the light reflected and transmitted by the fingerprint according to the above-described example embodiments may be formed to pass through the first and second layers 120a and 120b.

As an example implementation, the metal wirings 121a and 121b are respectively formed in various forms in the first and second layers 120a and 120b to prevent light from passing through a region except for the pinholes 122. For example, the metal wirings 121a formed in the first layer 120a may be disposed in parallel in a first direction, and the metal wirings 121b formed in the second layer 120b may be disposed in parallel in a second direction perpendicular to the first direction. According to the example embodiment illustrated in FIG. 5, a pinhole mask may not be formed as a separate layer in the fingerprint sensor, and the pinholes 122 may be formed in a plurality of layers in which the metal wirings 121a and 121b are formed.

For example, the metal wirings 121a and 121b may be additionally formed regardless of wirings for transmitting electrical signals for an actual operation of an image sensor. Also, the first and second layers 120a and 120b may be additionally provided in addition to a layer in which wirings for an actual operation of the image sensor are formed. Also, the device layer 130 in which PDs are formed may be disposed under the pinholes 122. In this case, since the pinholes 122 are implemented in a semiconductor process for implementing a fingerprint sensor, the pinholes 122 may be defined as being embedded in the fingerprint sensor.

Meanwhile, referring to FIG. 6, a fingerprint sensing system 200 may include a display panel 210, a pinhole mask layer 220, and a device layer 230. In this case, the pinhole mask layer 220 and the device layer 230 may constitute the fingerprint sensor according to an example embodiment of inventive concepts. Also, the display panel 210 may include a plurality of light sources similar to the above-described embodiment, and each of the light sources may be, for example, an OLED.

For example, the pinhole mask layer 220 may be implemented as a silicon wafer, a metal layer, or any material layer through which light does not pass, and may include a plurality of pinholes 221. Also, the device layer 230 may be an image sensor layer in which PDs are formed and may correspond to a component, which senses light passing through the plurality of pinholes 221 and generates an electrical signal. As described above, the pinhole mask layer 220 and the device layer 230 may be configured as different layers. According to one example embodiment, the pinhole mask layer 220 may be stacked on the device layer 230 in a semiconductor process for implementing the device layer 230. In one example embodiment, the pinhole mask layer 220 may be located to be aligned with the device layer 230.

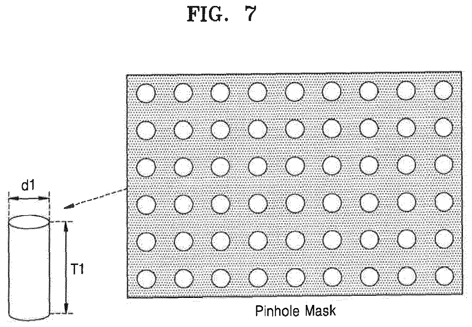

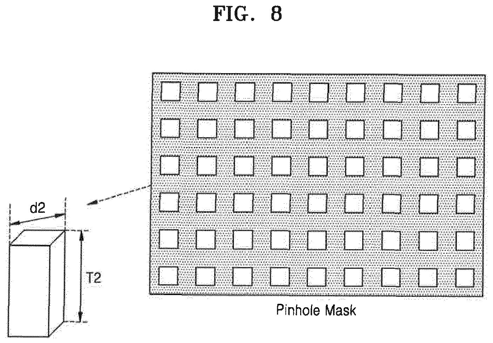

FIGS. 7 and 8 are diagrams illustrating an example implementation of a pinhole mask according to an example embodiment of inventive concepts.

Referring to FIG. 7, the pinhole mask may include a plurality of pinholes arranged in an array form, and a cross section of each of the pinholes may have a circular shape. A diameter d1 of each pinhole and a thickness T1 of the pinhole mask may be variously changed according to a determined angle of view.

FIG. 8 illustrates another example implementation of the pinhole mask. Referring to FIG. 8, the pinhole mask may include a plurality of pinholes arranged in an array form, and a cross section of each of the pinholes may have a rectangular shape. A diagonal line d2 of each pinhole and a thickness T2 of the pinhole mask may be variously changed according to a determined angle of view. Also, an example embodiment of inventive concepts are not limited to the examples of FIGS. 7 and 8, the pinholes formed in the pinhole mask may be implemented to be arranged to have a diamond shape or any polygonal shape in addition to a square shape in which adjacent pinholes are arranged at the same distance on the basis of one pinhole in up, down, left, and right directions, and a cross-section of each pinhole may also be implemented to have a hexagonal shape or any polygonal shape in addition to a circular shape, a square shape, or an elliptical shape.

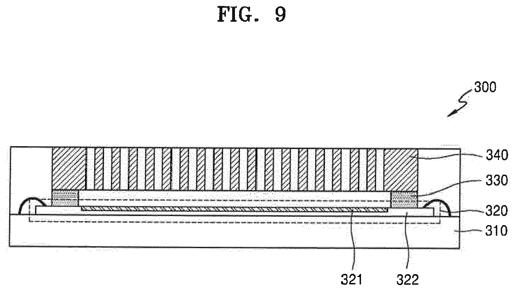

FIG. 9 is a diagram illustrating a structure of a fingerprint sensor package according to an example embodiment of inventive concepts. The fingerprint sensor package may be generated through a packaging process for the fingerprint sensor in the above-described embodiments. A fingerprint sensor package 300 illustrated in FIG. 9 may be attached to a display panel, and thus an on-display fingerprint sensor may be implemented. Also, the fingerprint sensor package 300 may be mounted on a board (not illustrated) as a sensor integrated circuit (IC), and image data corresponding to a fingerprint sensing result generated from the fingerprint sensor package 300 may be provided to an external device or system.

Referring to FIG. 9, the fingerprint sensor package 300 may include a package substrate 310, an image sensor 320 mounted on the package substrate 310, and a pinhole mask 340 located on the image sensor 320. The pinhole mask 340 may be implemented according to the above-described embodiments. For example, in the pinhole mask 340, a region except pinholes may be implemented as any material layer through which light does not pass, and a plurality of pinholes through which light reflected by a fingerprint passes may be formed. Also, a support 330 for supporting the pinhole mask 340 may be formed on the image sensor 320, and the pinhole mask 340 may be stacked and supported on the support 330.

According to the above structure, an air gap having a length corresponding to a height of the support 330 may be formed in the fingerprint sensor package 300. A distance between the image sensor 320 and the pinhole mask 340 may be adjusted according to the height of the air gap, and thus the height of the support 330 may correspond to a parameter which determines a diameter of the optical sensing region corresponding to a size of the sensor pixel.

The image sensor 320 may include a light-receiving region 321 which senses light transmitted through a plurality of pinholes of the pinhole mask 340, and a logic region 322 which generates image data through logic processing on an electrical signal from the light-receiving region 321. The light-receiving region 321 and the logic region 322 may be formed in different wafers (or separate semiconductor substrates). In this case, the light-receiving region 321 and the logic region 322 may be classified as separate chips. Alternatively, in another embodiment, the light-receiving region 321 and the logic region 322 may be implemented in one semiconductor chip.

The light-receiving region 321 may include the plurality of sensor pixels described in the above embodiments, and each of the sensor pixels may include one or more PDs. Since light is reflected by ridges or valleys of the fingerprint, a difference in an amount of light which is sensed in the sensor pixels may be generated, and the light-receiving region 321 provides electrical signals which are differently generated for each sensor pixel to the logic region 322. Logic circuits included in the logic region 322 may generate contrast or partial image data in units of a fingerprint pixel used to configure the entire fingerprint image by performing a processing operation such as an analog-digital conversion on the electrical signals, and an fingerprint image may be configured by using the contrast or partial image data. Also, the fingerprint image generated in this way may be provided to an external device or system through the package substrate 310.

Meanwhile, in the embodiment illustrated in FIG. 9, an externally mounted structure in which the pinhole mask 340 is stacked on the image sensor 320 in a packaging process for the image sensor 320 is illustrated. Accordingly, the pinhole mask 340 and the image sensor 320 may be manufactured through separate processes, and a process of assembling the pinhole mask 340 and the image sensor 320 into one package may be performed.

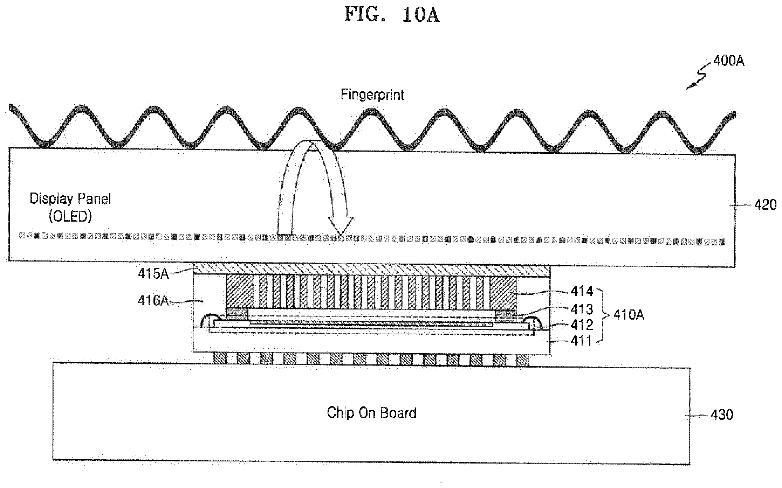

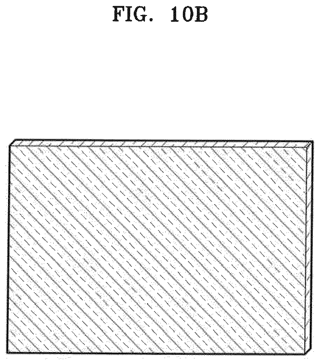

FIGS. 10A and 10B are diagrams illustrating an example in which a fingerprint sensor package is attached to a display panel in a fingerprint sensing system. Also, FIG. 10B illustrates an example implementation of an adhesive element for combining the fingerprint sensor package and the display panel illustrated in FIG. 10A. In some example embodiments, it is assumed that the fingerprint sensor package is implemented in the form of the package illustrated in FIG. 9.

Referring to FIG. 10A, a fingerprint sensing system 400A may include a fingerprint sensor package 410A, a display panel 420 to which the fingerprint sensor package 410A is attached, and a board 430 for mounting the fingerprint sensor package 410A.

The fingerprint sensor package 410A may include a package substrate 411, an image sensor 412 mounted on the package substrate 411, a support 413 formed on the image sensor 412, and a pinhole mask 414 including a plurality of pinholes. In the same or similar way as described above, an air gap may be formed between the image sensor 412 and the pinhole mask 414. The board 430 may correspond to a mother board such as a printed circuit board (PCB) of a smart phone and the like. As the fingerprint sensor package 410A is connected to the board 430, the fingerprint sensor package 410A may be mounted in the form of a chip on board (CoB). Also, image data may be provided to the board 430 through connection terminals formed on one surface of the fingerprint sensor package 410A, According to one example embodiment, the image data may be provided to the board 430 through a plurality of solder balls formed on one surface of the fingerprint sensor package 410A.

The fingerprint sensor package 410A may further include a molding 416A, and may be attached to the display panel 420 through an adhesive element 415A. An element capable of maintaining an adhesive force even in a change of temperature, humidity, or the like may be applied to the adhesive element 415A as an adhesive film or a liquid-type (or a resin) adhesive. For example, the adhesive element 415A may be implemented as an optical clear adhesive (OCA).

For example, the adhesive element 415A may be disposed on an upper surface of the fingerprint sensor package 410A. Alternatively, the adhesive element 415A may be disposed on one surface of the fingerprint sensor package 410A in a packaging process for the fingerprint sensor. In this case, the fingerprint sensor package 410A may be defined to further include the adhesive element 415A layer. The adhesive element 415A may serve as a buffer support when the fingerprint sensor package 410A is attached to the display panel 420, and a thickness of the adhesive element 415A may affect a distance between the fingerprint pixel and the pinhole. As the thickness of the adhesive element 415A is increased, an area of the fingerprint pixel seen by the pinhole may be increased, and the thickness of the adhesive element 415A may be adjusted as long as different fingerprint pixels do not overlap each other.

Meanwhile, referring to FIG. 10B, the adhesive element 415A such as an OCA or the like may be implemented using a transparent or translucent element. According to one example embodiment, the adhesive element 415A may be implemented in the form of a plane which covers an upper portion of the fingerprint sensor package 410A. That is, the adhesive element 415A may be implemented to cover a region in which the pinholes of the pinhole mask 414 are located, in the fingerprint sensor package 410A to bond the fingerprint sensor package 410A to the display panel 420. Also, a distance between the pinhole mask 414 and the fingerprint pixel may be adjusted according to the thickness of the adhesive element 415A, and thus the thickness of the adhesive element 415A may be included in a parameter which determines a diameter of the optical sampling region corresponding to a size of the fingerprint pixel.

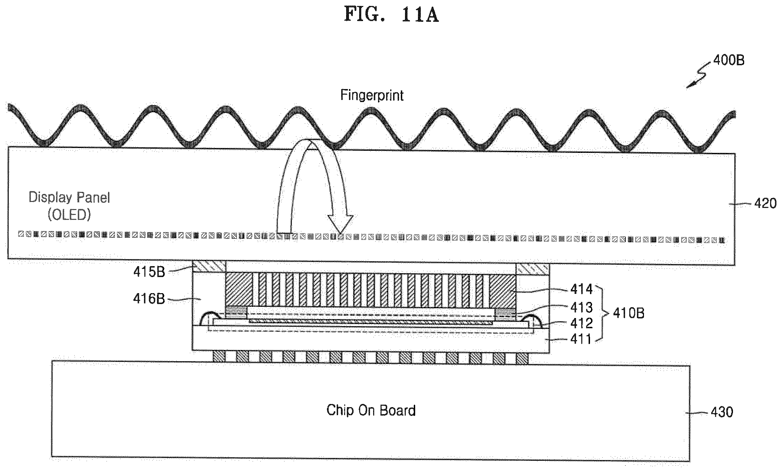

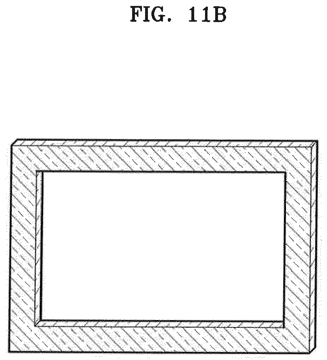

FIGS. 11A and 11B are diagrams illustrating another example in which a fingerprint sensor package is attached to a display panel in a fingerprint sensing system. Also, FIG. 11B illustrates another example implementation of an adhesive element for combining the fingerprint sensor package and the display panel illustrated in FIG. 11A. In description of a configuration of the fingerprint sensing system including the fingerprint sensor package illustrated in FIGS. 11A and 11B, a detailed description of the same configuration as that illustrated in FIGS. 10A and 10B will be omitted.

A fingerprint sensing system 400B includes a fingerprint sensor package 410B, and the fingerprint sensing system 400B may further include a display panel 420 to which the fingerprint sensor package 410B is attached and a board 430 for mounting the fingerprint sensor package 410B. Also, the fingerprint sensor package 410B may include a package substrate 411, an image sensor 412, a support 413, and a pinhole mask 414. Also, the fingerprint sensor package 410B may further include an adhesive element 415B and a molding 416B. According to one example embodiment, the adhesive element 415B may be disposed along an outer region of an upper surface of the fingerprint sensor package 410B, that is, an edge region thereof, to bond the fingerprint sensor package 410B to the display panel 420. Also, the pinhole mask 414, the support 413, the adhesive element 415B, and the like, as various components illustrated in FIG. 11A, may be implemented to have the same or similar thicknesses as those of the components illustrated in FIG. 10A.

Referring to FIG. 11B, the adhesive element 415B such as an OCA and the like may be implemented using a transparent, translucent, or opaque element. According to one example embodiment, as the adhesive element 415B is implemented in the form of a window frame, holes may be formed to correspond to regions in which pinholes are located. Accordingly, light reflected by a fingerprint may be provided to the image sensor 412 through an air gap (or an air layer) without passing through the adhesive element 415B, and the adhesive element 415B may be implemented using an opaque element. Also, since a transmittance of light passing through the air is higher than that of an adhesive element implemented using a transparent or translucent material, the sensitivity of the image sensor 412 for sensing the light reflected and transmitted by the fingerprint may be further increased.

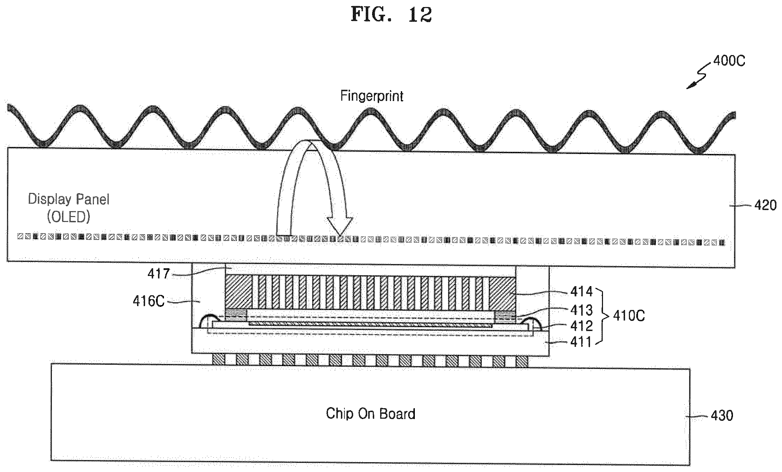

FIG. 12 is a diagram illustrating still another example in which a fingerprint sensor package is attached to a display panel in a fingerprint sensing system. In description of a configuration of the fingerprint sensing system including the fingerprint sensor package illustrated in FIG. 12, a detailed description of the same configuration as the configurations illustrated in FIGS. 10A and 10B will be omitted.

A fingerprint sensing system 400C includes a fingerprint sensor package 410C, and the fingerprint sensing system 400C may further include a display panel 420 to which the fingerprint sensor package 410C is attached and a board 430 for mounting the fingerprint sensor package 410C. Also, the fingerprint sensor package 410C may include a package substrate 411, an image sensor 412, a support 413, a pinhole mask 414, and a molding 416C. Also, the pinhole mask 414, the support 413, and the like, as various components illustrated in FIG. 12, may be implemented to have the same and similar thicknesses as those of the components illustrated in FIGS. 10A and 11A.

In the present embodiment, the adhesive element in the above-described embodiment may be removed from the fingerprint sensing system 400C. In this case, a height of the molding 416C included in the fingerprint sensor package 410C may be greater than those in the embodiments illustrated in FIGS. 10A and 11A. Accordingly, since a height of an edge region of the fingerprint sensor package 410C increases, the molding 416C located at the edge region of the fingerprint sensor package 410C may come in contact with one surface of the display panel 420. Also, as the height of the molding 416C increases, an air gap 417 may be formed between one surface of the display panel 420 and the pinhole mask 414, and light reflected by a fingerprint may be transmitted to the image sensor 412 through the air gap 417 and pinholes.

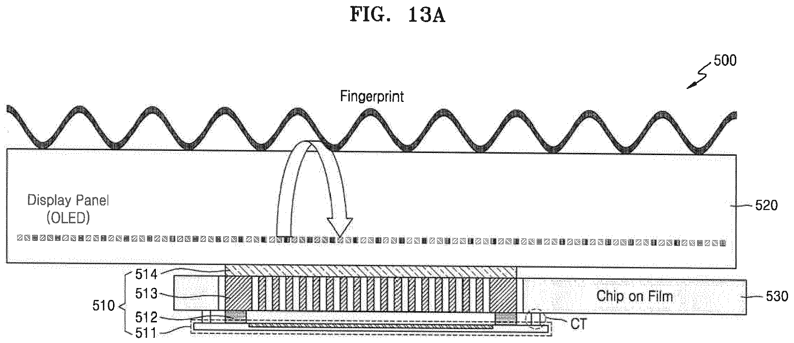

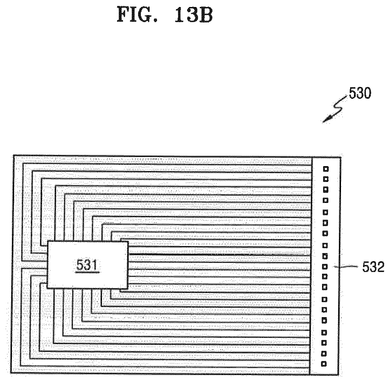

FIGS. 13A and 13B are structural diagrams illustrating another example implementation of the fingerprint sensing system. In FIGS. 13A and 13B, an example in which a fingerprint sensor package is mounted on a film in the form of a chip on film (COF) is illustrated.

Referring to FIGS. 13A and 13B, a fingerprint sensing system 500 includes a fingerprint sensor package 510, and the fingerprint sensing system 500 may further include a display panel 520 to which the fingerprint sensor package 510 is attached and a film 530 for mounting the fingerprint sensor package 510. The film 530 may include a wiring for interface. Also, the fingerprint sensor package 510 may include an image sensor 511, a support 512, a pinhole mask 513, and an adhesive element 514. Although not illustrated in FIG. 13A, the fingerprint sensor package 510 may further include a molding.

The film 530 may be implemented using a flexible element, and wirings for electrical transmitting of signals may be formed on at least one surface of the film 530. Also, wirings passing through the film 530 may be further formed in the film 530 to transmit signals from one surface of the film 530 to the other surface thereof. For example, when the fingerprint sensor package 510 is attached to the display panel 520, a surface of the film 530 facing the display panel 520 may be referred to as a first surface and the opposite surface as a second surface.

For example, the fingerprint sensor package 510 may be implemented in the same or similar manner as the fingerprint sensor package illustrated in the above-described embodiments. Also, although an example, in which the image sensor is mounted on one surface of the package substrate and the solder ball is formed on another surface of the package substrate to provide the image data to the board through the solder ball, is described in the above-described embodiments, an example in which the image data is provided to the film 530 including the wiring for interface instead of the package substrate is illustrated in the embodiment illustrated in FIG. 13A. Also, although a thickness of the pinhole mask 513 in the fingerprint sensor package 510 is illustrated as being the same as a thickness of the film 530 for convenience of illustration in FIG. 13A, the thickness of the pinhole mask 513 may be smaller than that of the film 530 or the thickness of the pinhole mask 513 may be greater than that of the film 530.

Meanwhile, referring to FIG. 13B, a hole 531 having a size enough to allow the fingerprint sensor package 510 to pass therethrough may be formed in the film 530, and the fingerprint sensor package 510 may be attached to the display panel 520 through the hole 531 formed in the film 530. According to one example embodiment, the hole 531 may be formed in the film 530 so that an upper portion (e.g., a region at which the pinhole mask 513 is located) of the fingerprint sensor package 510 may pass therethrough, and the upper portion of the fingerprint sensor package 510 may be attached to the display panel 520 through the hole 531 of the film 530. Also, according to one example embodiment, the fingerprint sensor package 510 may be attached to the display panel 520 through the adhesive element 514, and the adhesive element 514 may be implemented in various forms (e.g., a plane form and a window frame form) according to the above-described embodiments. Also, the adhesive element 514 may be implemented as a transparent or translucent element.

Although a size of the adhesive element 514 is illustrated as being the same as a size of an upper surface of the fingerprint sensor package 510 for convenience of illustration in FIG. 13A, inventive concepts are not limited thereto. For example, according to one example embodiment, the adhesive element 514 may be implemented to be greater than the hole 531, and an edge region of the adhesive element 514 may be attached to the display panel 520 while being attached to a periphery of the hole 531 of the film 530.

Meanwhile, signals generated in the fingerprint sensor package 510 may be transmitted to an external device or system through the plurality of wirings in the film 530. For example, the signals (e.g., image data) generated from the fingerprint sensor package 510 may be transmitted to the second surface of the film 530. As an example implementation, a connection terminal ct may be disposed on a logic region of the image sensor 511, and electrically connected to wirings formed in the second surface of the film 530. Connectors 532 for electrically connecting to other devices or systems may be provided in the film 530. For example, the image data generated from the fingerprint sensor package 510 may be transmitted to an external device or system mounted on a main board (not illustrated) through the connectors 532.

According to the above-described embodiment, since a transmission path of the image data generated from the fingerprint sensor package 510 may be flexibly implemented through the film 530, ease of arrangement of the devices in the fingerprint sensing system 500 may be improved. Also, the entire thickness of the fingerprint sensor package 510 mounted in the form of a COF on the film 530 may be reduced.

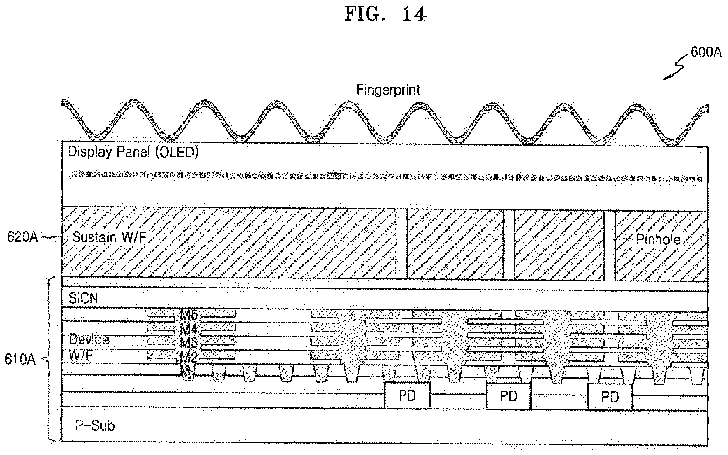

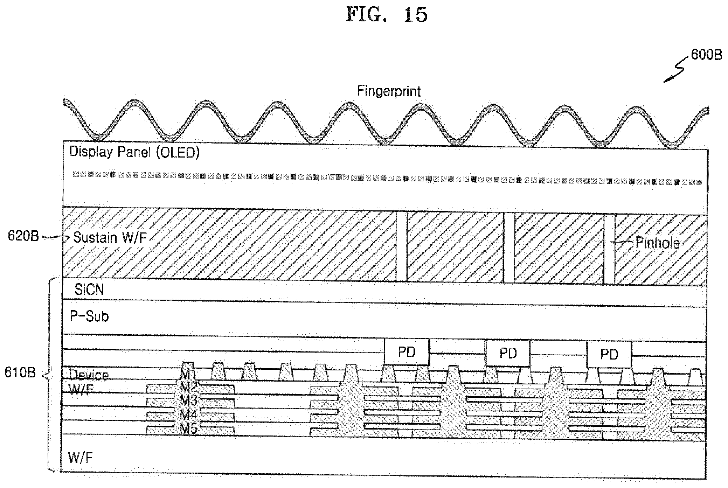

FIGS. 14 and 15 are diagrams illustrating an example implementation of a fingerprint sensor in which a pinhole mask is embedded in an image sensor. For example, in a semiconductor process for implementing an image sensor, holes may be formed in at least one layer in a form same as or similar to the pinholes in the above-described embodiment, and the layer in which the holes are formed may be stacked on an upper portion of a layer in which PDs are formed. For example, since a sustain wafer in which a plurality of holes are formed may perform a function of the pinhole mask in the above-described embodiment, the sustain wafer may be referred to as a pinhole mask in the following embodiments.

FIG. 14 is a cross-sectional view illustrating a front side illumination (FSI) type fingerprint sensor according to one example embodiment of inventive concepts.

Referring to FIG. 14, a fingerprint sensor 600A according to the present embodiment may be implemented using a FSI method. Specifically, the fingerprint sensor 600A may include a plurality of wafers for implementing an image sensor and a pinhole mask. For example, the fingerprint sensor 600A may include a device wafer 610A in which an image sensor is implemented and a sustain wafer 620A located on the device wafer 610A. A plurality of pinholes may be formed in the sustain wafer 620A to correspond to a region in which sensor pixels (or PDs) are formed in the device wafer 610A. For example, each of the plurality of pinholes may be disposed to be aligned with a center of each of the sensor pixels implemented in the device wafer 610A.

As an example implementation, the device wafer 610A may include a semiconductor substrate (e.g., P- Sub) and one or more layers formed on the semiconductor substrate. For example, one or more epitaxial layers growing as the same crystal structure as the semiconductor substrate may be formed.

One or more photoelectric conversion regions for sensing light reflected by a fingerprint in the image sensor may be formed in the epitaxial layers. For example, the PDs in the above-described embodiment may be formed in the photoelectric conversion regions through an ion implantation process. As a modified embodiment, various types of photoelectric conversion elements such as a phototransistor, a photo gate, a pinned PD, and the like may be formed in the photoelectric conversion regions.

Meanwhile, since the fingerprint sensor is implemented using a FSI method, one or more metal layers may be formed on the epitaxial layer in which the PDs are formed. In FIG. 14, an example in which five metal layers M1 to M5 are formed is illustrated, and a dielectric for electrical insulation between the metal layers M1 to M5 may be implemented. As an example implementation, the dielectric may be formed in the form of an inter-layer dielectric or inter level dielectric (ILD) or an intermetal dielectric (IMD), and an oxide or nitride-based insulating device may be used as a material of the dielectric. Also, metal lines and contacts may be formed in each of the metal layers M1 to M5.

Meanwhile, the sustain wafer 620A may be disposed on the metal layers M1 to M5. The sustain wafer 620A may be used for holding or supporting an epitaxial layer. According to example embodiments of inventive concepts, the pinholes formed in the sustain wafer 620A may form a focus of light reflected and transmitted by a fingerprint. The fingerprint sensor 600A including the device wafer 610A and the sustain wafer 620A may be packaged and attached to one surface of the display panel, and a packaging process of the fingerprint sensor 600A may be performed in the same manner as the packaging processes illustrated in the above-described embodiments. Also, various mounting methods may be applied to the packaged fingerprint sensor 600A. For example, the packaged fingerprint sensor 600A may be mounted on the board in the form of a COB, or the packaged fingerprint sensor 600A may be mounted on the film in the form of a COF. Although the display panel is illustrated as being disposed on the sustain wafer 620A for convenience of illustration in FIG. 14, the packaged fingerprint sensor 600A may be substantially attached to the display panel through the adhesive elements in the above-described embodiments.

Also, an example in which at least one layer is further disposed between the metal layers M1 to M5 and the sustain wafer 620A is illustrated in FIG. 14. For example, one or more layers implemented with silicon carbon nitride (SiCN) or the like are disposed, and thus the sustain wafer 620A may be stacked thereon. Also, for example, the transparency of the sustain wafer 620A may be variously adjusted. According to the above-described embodiments, a material for reducing a transmittance thereof may be applied to a wafer having a silicon material to implement the sustain wafer 620A.

According to the fingerprint sensor 600A implemented as described above, the sustain wafer 620A corresponding to the pinhole mask may be stacked on the device wafer 610A in a process for implementing the image sensor. Accordingly, in a packaging process for the image sensor, a process of stacking a separate pinhole layer on the image sensor may be omitted. Also, in the packaged fingerprint sensor package, since it is not necessary to provide a separate holder for fixing a pinhole layer, the entire thickness of the fingerprint sensor package may be reduced.

FIG. 15 is a cross-sectional view illustrating a back side illumination (BSI) type fingerprint sensor according to one example embodiment of inventive concepts. In description of a structure of a fingerprint sensor 600B illustrated in FIG. 15, a detailed description of the same configuration as that illustrated in FIG. 14 will be omitted.

Referring to FIG. 15, the fingerprint sensor 600B according to the present embodiment may be implemented using a BSI method. For example, the fingerprint sensor 600B may include a device wafer 610B in which an image sensor is implemented and a sustain wafer 620B located on the device wafer 610B. Also, a plurality of pinholes may be formed in the sustain wafer 620B.

As an example implementation, since the image sensor in the fingerprint sensor 600B is implemented using a BSI method, a layer (e.g., an epitaxial layer) in which PDs are formed in the image sensor may be located between the sustain wafer 620B and the metal layers M1 to M5. Accordingly, light, which is reflected by a fingerprint and passes through the pinholes of the sustain wafer 620B, may be provided to the PDs without passing through the metal layers M1 to M5. Also, a separate wafer for a function same as or similar to a supporting or holding function of the above-described sustain wafer may be disposed under the metal layers M1 to M5 in the drawing.

Similar to the above-described embodiment, the plurality of pinholes of the sustain wafer 620B may form a focus of light reflected and transmitted by a fingerprint. In the embodiment illustrated in FIG. 15, since the image sensor is implemented using a BSI method, distances between the pinholes of the sustain wafer 620B and the PDs may be smaller than those in the embodiment illustrated in FIG. 14, and thus a method of implementing an image sensor such as a FSI method, a BSI method, or the like may affect a size of the sensor pixel.

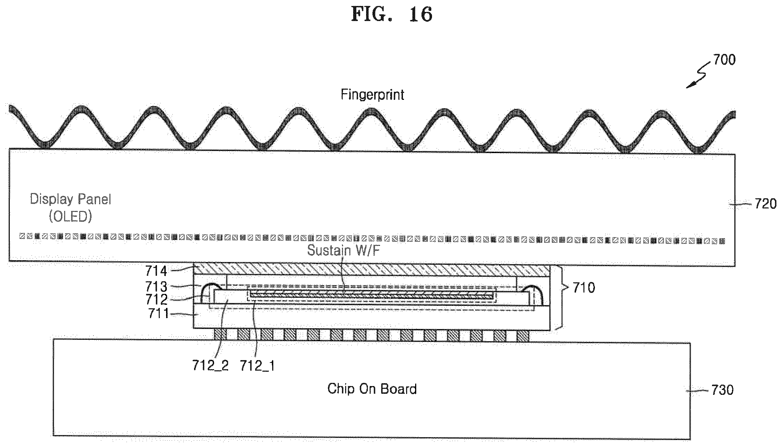

FIG. 16 is a diagram illustrating an example in which a fingerprint sensor package including a pinhole layer having an embedded structure is attached to a display panel. Although an example in which an adhesive element has a plane form and a fingerprint sensing system is mounted in a COB form is illustrated in FIG. 16, as described above, a fingerprint sensing system to which a pinhole layer having an embedded structure is applied may be implemented by various other methods.

Referring to FIG. 16, a fingerprint sensing system 700 may include a fingerprint sensor package 710, a display panel 720, and a board 730. Also, the fingerprint sensor package 710 may include a package substrate 711, an image sensor 712 mounted on the package substrate 711, and a molding 713. Also, the fingerprint sensor package 710 may be attached to the display panel 720 through an adhesive element 714.

The image sensor 712 may include a light-receiving region 712_1 and a logic region 712_2. The light-receiving region 712_1 may be implemented according to the above-described embodiment illustrated in FIG. 14 or 15, and thus the light-receiving region 712_1 may include a sustain wafer, which is stacked on PDs in an image sensor process and has a plurality of pinholes. Also, the logic region 712_2 may generate image data through logic processing on an electrical signal from the light-receiving region 712_1 and provide the generated image data to the board 730 through the package substrate 711. In the above-described structure, according to the present embodiment, a process of stacking a pinhole mask on the image sensor 712 may be omitted in a packaging process.

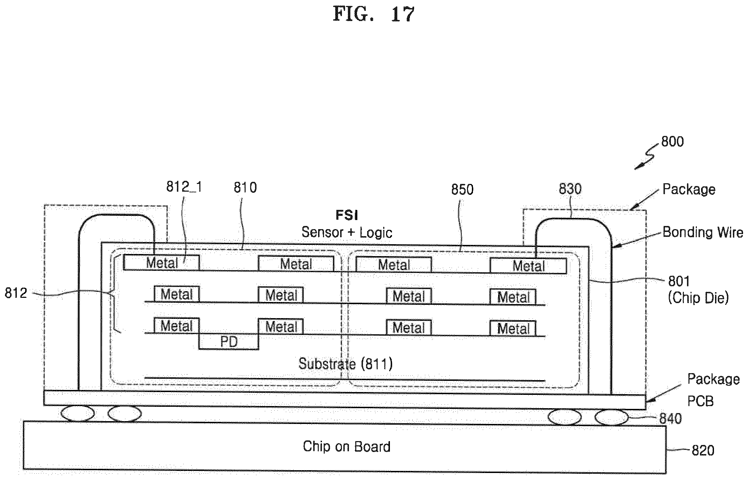

FIGS. 17 to 24 are diagrams illustrating various structures of a fingerprint sensor package according to at least some example embodiments of inventive concepts. Although not illustrated in FIGS. 17 to 24 for convenience of description, the pinhole mask according to the above-described example embodiments may be disposed on the image sensor in an externally mounted form in the fingerprint sensor package, or a layer for performing a pinhole mask function which may be implemented as the above-described sustain wafer or the like may be embedded in the image sensor.

The image sensor included in the fingerprint sensor may include a light-receiving region, which senses light reflected by a fingerprint and generates an electrical signal corresponding thereto, and a logic region which generates image data through logic processing on the electrical signal from the light-receiving region. According to one example embodiment, the light-receiving region and the logic region may be manufactured as separate chips (e.g., chips in a die unit) on different semiconductor substrates, and a sensor chip including the light-receiving region and a logic chip including the logic region may be packaged in one semiconductor package. Alternatively, the light-receiving region and the logic region may be manufactured on one semiconductor substrate and packaged in an image sensor chip. According to the above-described embodiments, a separate pinhole mask (not illustrated) may be stacked on the sensor chip in the packaging process. Alternatively, according to the above-described embodiments, in a semiconductor process for manufacturing a sensor chip, a sustain wafer in which pinholes are formed may be stacked on PDs.

Hereinafter, for convenience of description, in description of features of example embodiments of inventive concepts, an example in which the light-receiving region and the logic region are manufactured on the same substrate may be referred. Accordingly, a chip in which the light-receiving region (or a sensor region) and the logic region are manufactured on the same semiconductor substrate may be referred to as an image sensor chip. However, example embodiments of inventive concepts are not limited to such terms. For example, a chip including both the sensor region and the logic region may be referred to as a sensor chip.

Referring to a fingerprint sensor package 800 of FIG. 17, an image sensor chip 801 may be mounted on a package substrate Package PCB. Since a sensor region 810 and a logic region 850 which are included in the image sensor chip 801 does not have a stacked structure, one or more layers may be located on the same plane. Alternatively, when the sensor region 810 and the logic region 850 are manufactured as separate chips, the chips may be mounted on the package substrate Package PCB on the same plane. Also, an example in which the sensor region 810 and the logic region 850 are electrically connected to the package substrate through bonding wires 830 is illustrated in the embodiment of FIG. 17.

Meanwhile, an example in which the sensor region 810 is implemented by a FSI method in the above-described example embodiment is illustrated in the example embodiment of FIG. 17, and thus the sensor region 810 may include a plurality of PDs formed in a semiconductor substrate 811 and one or more metal layers 812 on which metal lines 812_1 are formed. Also, light reflected by a fingerprint may be transmitted to the PDs through a pinhole layer (not illustrated) and the metal layers 812.

The fingerprint sensor package 800 may be mounted in the form of a CoB according to the above-described embodiment. Connection terminals for transmitting signals between the sensor region 810 and the logic region 850 in the fingerprint sensor package 800 and a board 820 may be formed on one surface of the package substrate, and an example in which a plurality of solder balls 840 are attached to one surface of the package substrate is illustrated in the embodiment of FIG. 17.

Meanwhile, since the sensor region 810 is implemented by a FSI method, a pad for electrically connecting to the outside may be present at a top of the sensor region 810, and in the sensor region 810, the metal lines 812_1 located thereon and the package substrate may be electrically connected through the pad. For example, the pad of the sensor region 810 and the package substrate may be electrically connected through one of the bonding wires 830. The electrical signal from the sensor region 810 in the fingerprint sensor package 800 may be provided to the logic region 850, and the image data from the logic region 850 may be provided to an external system through the solder balls 840 and the board 820.

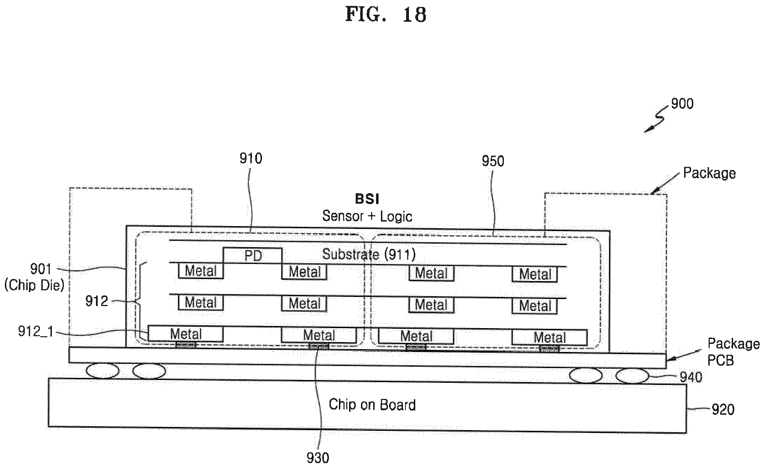

FIG. 18 illustrates another example in which the image sensor chip is mounted on the package substrate. In an image sensor chip 901 illustrated in FIG. 18, an example in which a sensor region 910 is implemented by a BSI method and a fingerprint sensor package 900 is mounted in a CoB form according to the above-described embodiment is illustrated.

Referring to FIG. 18, the image sensor chip 901 of the fingerprint sensor package 900 includes the sensor region 910 and a logic region 950. Since the sensor region 910 is implemented by a BSI method, a semiconductor substrate 911 in which a plurality of PDs are formed may be located on the sensor region 910, and one or more metal layers 912 in which metal lines 912_1 are formed may be located under the PDs. Also, a pad may be located at a bottom of the sensor region 910. For example the sensor region 910 may be electrically connected to one surface of the package substrate through the pad and a connection terminal (e.g., a bump 930). Also, an electrical signal from the sensor region 910 may be transmitted to the board 920 through the bump 930 and a connection terminal (e.g., a solder ball 940) attached to another surface of the package substrate.

According to the above-described embodiments, since the sensor region and the logic region do not have a stacked structure in the fingerprint sensor package, a thickness of the fingerprint sensor package may be reduced. Also, as illustrated in FIG. 18, in the embodiment in which a BSI method is applied, since the sensor region 910 may be connected to the package substrate through the bump 930 without using wire bonding, a thickness of the fingerprint sensor package 900 may be prevented from being increased due to the sensor region 910.

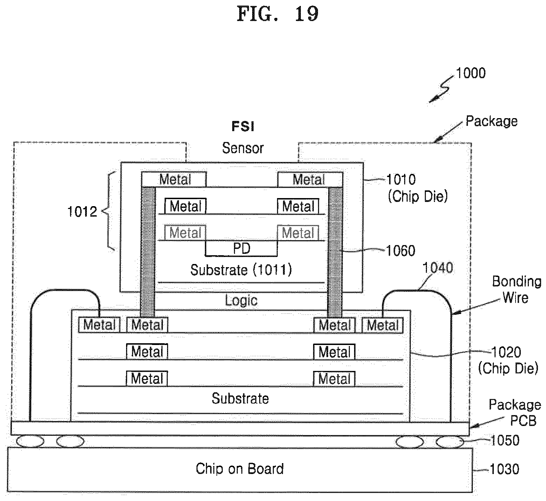

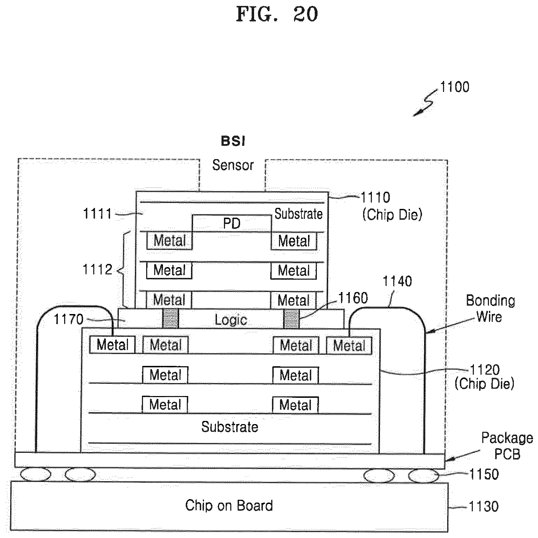

FIGS. 19 and 20 are block diagrams illustrating an example implementation of a fingerprint sensor package having a structure in which a sensor chip and a logic chip are stacked. In FIGS. 19 and 20, an example in which the sensor chip and the logic chip are manufactured on separate semiconductor substrates is illustrated. Also, while an example in which the sensor chip is implemented by a FSI method is illustrated in FIG. 19, an example in which the sensor chip is implemented by a BSI method is illustrated in FIG. 20. Also, as described above, although the pinhole mask according to example embodiments of inventive concepts are omitted in FIGS. 19 and 20 for convenience of illustration, the pinhole mask may be implemented in an externally mounted or embedded manner according to the above-described embodiments and provided in the fingerprint sensor package.

Referring to FIG. 19, a fingerprint sensor package 1000 may include a sensor chip 1010 in which PDs are formed and a logic chip 1020 in which logic circuits are formed, and the sensor chip 1010 and the logic chip 1020 may have a stacked structure in the fingerprint sensor package 1000. An example in which the sensor chip 1010 is implemented by a FSI method is illustrated in FIG. 19. Accordingly, a semiconductor substrate 1011 in which a plurality of PDs are formed may be located at a lower portion of the sensor chip 1010, and one or more metal layers 1012 may be located at an upper portion of the sensor chip 1010.

According to one example embodiment, in the fingerprint sensor package 1000, the sensor chip 1010 may be stacked on the logic chip 1020. The sensor chip 1010 may sense light reflected by a fingerprint to generate an electrical signal, and the electrical signal may be provided to the logic chip 1020 through a metal line formed in an upper metal layer of one or more metal layers 1012 in the sensor chip 1010. According to one example embodiment, a back via stack (BVS) 1060 passing through the semiconductor substrate 1011 of the sensor chip 1010 from at least one metal layer of the sensor chip 1010 may be formed in the sensor chip 1010, and the electrical signal from the sensor chip 1010 may be provided to the logic chip 1020 through the BVS 1060.

Meanwhile, the logic chip 1020 may include various circuits for processing the electrical signal to generate image data, and a plurality of metal layers for implementing various logic circuits. The electrical signal transmitted through the BVS 1060 may be provided to at least one metal layer in the logic chip 1020.

The logic chip 1020 may be mounted on the package substrate Package PCB and electrically connected to the package substrate. According to one example embodiment, one or more metal layers may be stacked on the semiconductor substrate of the logic chip 1020, and thus electrically connected to the package substrate through the pad located at a top of the logic chip 1020. For example, the logic chip 1020 may be electrically connected to the package substrate through the pad and a bonding wire 1040.

The fingerprint sensor package 1000 may be mounted on a board 1030 in a CoB form. For example, the logic chip 1020 may be connected to the board 1030 through solder balls 1050. Also, the logic chip 1020 may provide image data to an external system (e.g., a device for reconfiguring fingerprints) through the package substrate, the solder balls 1050, and the board 1030.

According to the embodiment illustrated in FIG. 19, when the sensor chip 1010 in which PDs are formed and the logic chip 1020 in which logic circuits are formed have a stacked structure and the sensor chip 1010 is implemented by a FSI method, since the sensor chip 1010 and the logic chip 1020 may be connected through the BVS 1060, an increase of a thickness of the fingerprint sensor package 1000 in the stacked structure of the sensor chip 1010 and the logic chip 1020 may be minimized. Meanwhile, since the sensor chip 1010 is stacked on the logic chip 1020 even when the logic chip 1020 is connected to the package substrate through the bonding wire 1040, an increase in the thickness of the fingerprint sensor package 1000 due to the bonding wire 1040 itself may not occur.

Also, according to the stacked structure of the sensor chip 1010 and the logic chip 1020, since the fingerprint sensor package 1000 recognizes the entire fingerprint of the finger, an area of the sensor chip 1010 which should sense light reflected by the fingerprint may be relatively large, but the area of the fingerprint sensor package 1000 may be prevented from increasing due to logic circuits other than the sensor chip 1010.

Meanwhile, referring to FIG. 20, a fingerprint sensor package 1100 may include a sensor chip 1110 having a stacked structure and a logic chip 1120 in which logic circuits are formed, and the sensor chip 1110 may be implemented by a BSI method. Accordingly, a semiconductor substrate 1111 in which a plurality of PDs are formed may be located on the sensor chip 1110, and one or more metal layers 1112 in which metal lines are formed may be located under the semiconductor substrate 1111.

Meanwhile, the logic chip 1120 may include various circuits for processing an electrical signal from the sensor chip 1110 to generate image data, and a plurality of metal layers for implementing the various circuits may be stacked on the semiconductor substrate. Also, the logic chip 1120 may be mounted on the package substrate, and the fingerprint sensor package 1100 may be connected to a board 1130 through a solder ball 1150. Also, a pad of the logic chip 1120 may be present at a top, and the logic chip 1120 and the package substrate may be electrically connected to each other through a bonding wire 1140.

Meanwhile, when the sensor chip 1110 is implemented by a BSI method, the metal layers 1112 of the sensor chip 1110 are located at a lower portion thereof, whereas the metal layers of the logic chip 1120 are located at an upper portion thereof. In this case, a metal line (e.g., a metal line of a lowermost metal layer) of the sensor chip 1110 and a metal line (e.g., a metal line of an uppermost metal layer) of the logic chip 1120 may be electrically connected through a through silicon via (TSV) 1160. For example, a separate layer 1170 such as an interposer and the like may be disposed between the sensor chip 1110 and the logic chip 1120, and an electrical signal generated in the sensor chip 1110 may be provided to at least one metal layer in the logic chip 1120 through the TSV 1160 passing through the separate layer 1170.

According to the embodiment illustrated in FIG. 20, the sensor chip 1110 and the logic chip 1120 may be electrically connected through the TSV 1160 without using bonding wires and thus an increase of a thickness in the stacked structure of the sensor chip 1110 and the logic chip 1120 may be minimized.

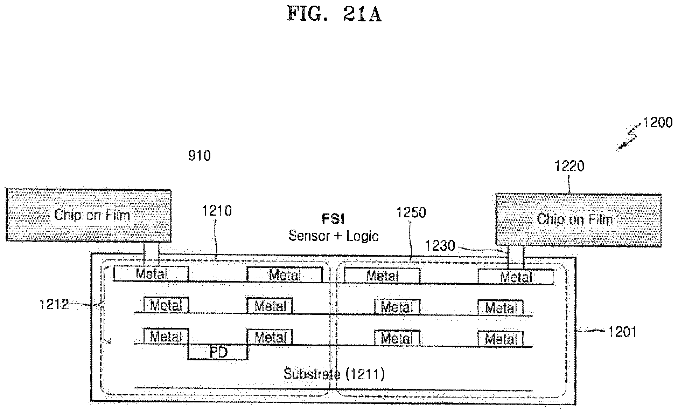

FIGS. 21A to 24 are block diagrams illustrating example implementations in which a fingerprint sensor package is mounted in a COF form. When a COF form is applied to the fingerprint sensor package, the fingerprint sensor package may be directly connected to a film through a connection terminal such as a bump or the like. That is, in embodiments illustrated in FIGS. 21A to 24, sensor chips or logic chips may be directly connected to a film through one or more connection terminals without being mounted on a separate package substrate.

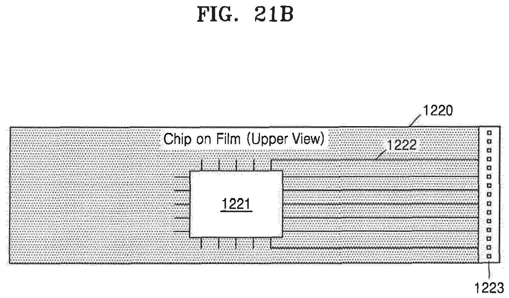

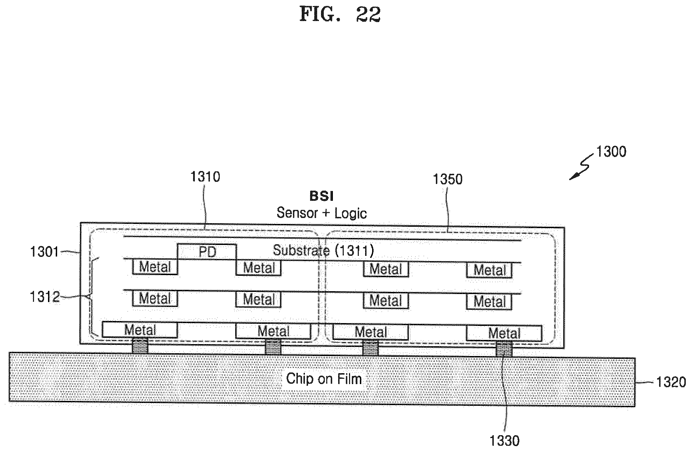

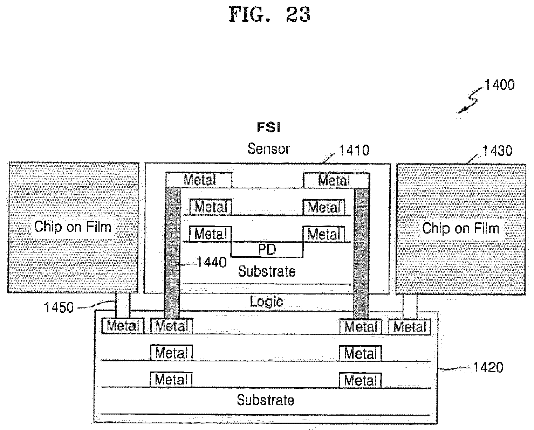

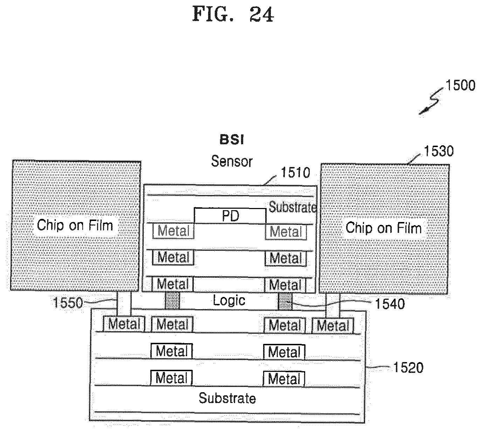

Meanwhile, a case in which a sensor region and a logic region are manufactured on the same semiconductor substrate is illustrated in FIGS. 21A, 21B, and 22, and a case in which a sensor chip and a logic chip have a stacked structure in a semiconductor package is illustrated in FIGS. 23 and 24.

Referring to FIG. 21A, an image sensor chip 1201 of a fingerprint sensor package 1200 may include a sensor region 1210 and a logic region 1250, and the sensor region 1210 may be implemented by a FSI method. The sensor region 1210 may include a plurality of PDs formed in a semiconductor substrate 1211 and one or more metal layers 1212 stacked thereon. Also, a pad may be formed at a top of the image sensor chip 1201, and thus the sensor region 1210 may be electrically connected to a film 1220 through a connection terminal such as a bump and the like. The sensor region 1210 may sense light transmitted through the metal layers 1212, and provide an electrical signal based on a sensing result to the film 1220.

In such an embodiment, the sensor region 1210 may be attached to a display panel (not illustrated) while being located under the film 1220, and thus light reflected by a fingerprint may be provided to the PDs through the film 1220 and one or more metal layers 1212. According to one example embodiment, as illustrated in FIG. 21B, a hole 1221 may be formed in a region (or a region through which light passes) corresponding to the PDs in the film 1220. The fingerprint sensor package 1200 may be connected to the film 1220 so that the PDs formed on the semiconductor substrate 1211 are located in a region corresponding to the hole 1221 formed in the film 1220. Also, in order to transmit a signal through the film 1220, wirings 1222 may be formed on at least one surface of the film 1220, and wiring lines passing through both surfaces of the film 1220 may be formed. In addition, the film 1220 may include connectors 1223 for connecting to other devices or systems.

Meanwhile, referring to FIG. 22, an image sensor chip 1301 of a fingerprint sensor package 1300 may include a sensor region 1310 and a logic region 1350 and the sensor region 1310 may be implemented by a BSI method.

Since the sensor region 1310 is implemented by a BSI method, a semiconductor substrate 1311 in which a plurality of PDs are formed may be located on the image sensor chip 1301, and one or more metal layers 1312 in which metal lines are formed may be located under the semiconductor substrate 1311. Also, a pad of the image sensor chip 1301 may be located at a lowermost end, and thus one or more lower metal lines of the sensor region 1310 may be electrically connected to a film 1320 through the pad and a connection terminal (e.g., a bump 1330). According to one example embodiment, the sensor region 1310 may be attached to a display panel (not illustrated) while being located on the film 1320, and thus light reflected by a fingerprint may be directly provided to the PDs without passing through the film 1320 or the metal layers 1312.

According to the embodiment illustrated in FIG. 22, since the light reflected by the fingerprint is provided to the PDs without passing through the film 1320, the film 1320 need not have a separate hole for a light propagation path.

In FIGS. 23 and 24, example implementations in which a sensor chip and a logic chip have a stacked structure and a fingerprint sensor package to which a COF method is applied are illustrated.

Referring to FIG. 23, a fingerprint sensor package 1400 may include a sensor chip 1410 and a logic chip 1420 and the sensor chip 1410 may be implemented by a FSI method. Also, the logic chip 1420 may include a plurality of metal layers in order to implement a logic circuit for logic processing an electrical signal from the sensor chip 1410 to generate image data, and the sensor chip 1410 and the logic chip 1420 may be electrically connected to each other through a BVS 1440 due to such a structure.

Meanwhile, in the fingerprint sensor package 1400, the logic chip 1420 may be electrically connected to a film 1430, and thus the logic chip 1420 may generate image data based on an electrical signal from the sensor chip 1410 to transmit the generated image data to the film 1430. For example, the plurality of metal layers of the logic chip 1420 may be stacked on a semiconductor substrate, a pad is located at a top of the logic chip 1420, and thus a metal line formed in at least one metal layer (e.g., a top metal layer) may be connected to the film 1430 through a connection terminal (e.g., a bump 1450). That is, the logic chip 1420 may receive the electrical signal from the sensor chip 1410 through the BVS 1440 and a metal line formed in a top metal layer and provide the electrical signal to lower metal layers, and image data generated by processing the electrical signal may be transmitted to the film 1430 through the metal line formed in the top metal layer and a bump 1450.

According to the embodiment illustrated in FIG. 23, a hole having an area so that the sensor chip 1410 may pass therethrough may be formed in the film 1430, and the fingerprint sensor package 1400 may have a structure in which the sensor chip 1410 is inserted into the hole of the film 1430. Accordingly, in mounting the fingerprint sensor package 1400 having a structure in which the sensor chip 1410 and the logic chip 1420 are stacked on the film 1430, the entire thickness thereof may be reduced.

Meanwhile, referring to FIG. 24, a fingerprint sensor package 1500 may include a sensor chip 1510 and a logic chip 1520 and the sensor chip 1510 may be implemented by a BSI method. Also, the logic chip 1520 may include a plurality of metal layers for logic processing an electrical signal from the sensor chip 1510 to generate image data, and the sensor chip 1510 and the logic chip 1520 may be electrically connected to each other through a TSV 1540 due to such a structure. Although not illustrated in FIG. 24, a separate layer (not illustrated) such as an interposer or the like in which the TSV 1540 is formed may be disposed between the sensor chip 1510 and the logic chip 1520.

Meanwhile, in the above-described embodiment, a metal line formed in at least one metal layer (e.g., a top metal layer) of the logic chip 1520 may be electrically connected to a film 1530 through a bump 1550. Also, a hole having an area so that the sensor chip 1510 may pass therethrough may be formed in the film 1530, and the fingerprint sensor package 1500 may have a structure in which the sensor chip 1510 is inserted into the hole of the film 1530.

Meanwhile, in the above-described embodiments, although an example in which the hole is formed in the film when the fingerprint sensor package having a stacked structure is connected to the film is described, inventive concepts are not limited thereto. For example, as illustrated in FIG. 22 described above, the above-described fingerprint sensor package having a stacked structure may be connected to the film in which a hole is not formed. In this case, when a region in which the sensor chip is located corresponds to an upper region of the fingerprint sensor package, the upper region of the fingerprint sensor package may be attached to one surface of the display panel.

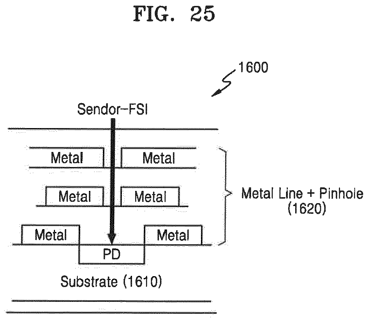

FIG. 25 is a diagram illustrating a fingerprint sensor according to an example embodiment of inventive concepts. In FIG. 25, a chip including a plurality of layers for implementing a sensor chip is illustrated as a component included in a fingerprint sensor 1600. According to the above-described embodiments, a logic chip (not illustrated) in which a logic circuit is formed may be further provided in the fingerprint sensor 1600.

The fingerprint sensor 1600 may include a semiconductor substrate 1610 in which a PD is formed and one or more metal layers 1620 stacked on the semiconductor substrate 1610. Also, a dielectric formed in the form of an ILD or an IMD may be disposed between the metal layers 1620, and each of the metal layers may include metal lines and contacts. Also, the dielectric may be implemented with a transparent or translucent material through which light may pass.

According to one example embodiment, a function of a pinhole may be implemented through the arrangement of the metal lines in the metal layers 1620 without a pinhole layer separately formed in the fingerprint sensor 1600. For example, when the sensor chip of the fingerprint sensor 1600 is implemented by a FSI method, light reflected by a fingerprint may be transmitted to the PD through the metal layers 1620. In this case, since the metal lines formed in the metal layers are implemented with a material through which light does not pass, the light may be transmitted through a space between the metal lines. According to the above-described embodiment, a diameter and height of the pinhole may serve as parameters which determine sizes of the fingerprint pixel and sensor pixel, and the metal lines may be formed so that a function (e.g., a function of transmitting the brightness of the reflected light to the PD) similar to the pinhole in the above-described embodiments is performed by the space between the metal lines.

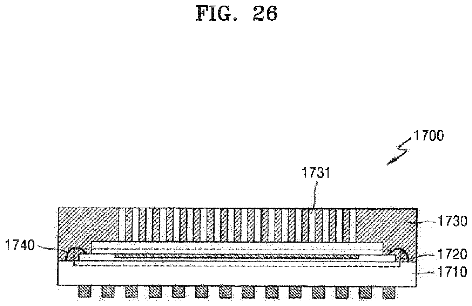

FIG. 26 is a diagram illustrating a structure of a fingerprint sensor package according to at least one example embodiment of inventive concepts.

Referring to FIG. 26, a fingerprint sensor package 1700 may include a package substrate 1710, an image sensor 1720 mounted on the package substrate 1710, and a molding 1730 corresponding to a package cover. Also, the image sensor 1720 may include a sensor chip and a logic chip, and the image sensor 1720 may be electrically connected to the package substrate 1710 through a connecting element such as a bonding wire or the like.

According to one example embodiment, in a molding 1730 of the fingerprint sensor package 1700, one or more holes 1731 may be formed in a region located on the image sensor 1720. For example, a thickness of the molding 1730 located on the fingerprint sensor package 1700 may have a value suitable for forming a focus of light according to the above-described embodiments, and a region in which the holes 1731 are formed in the molding 1730 may correspond to a region in which PDs (not illustrated) are formed in the image sensor 1720. According to one example embodiment, the molding 1730 may be implemented using an element having a low transmittance. For example, the molding 1730 may be implemented with an epoxy resin such as an epoxy molding compound (EMC).

The fingerprint sensor package 1700 implemented as illustrated in FIG, 26 may be attached to a display panel in various methods. For example, the fingerprint sensor package 1700 may be attached to the display panel through a transparent or translucent adhesive element having a plane form. Alternatively, for example, the fingerprint sensor package 1700 may be attached to the display panel through a transparent, translucent, or opaque adhesive element having a window frame form. Alternatively, for example, a height of an edge region in the molding 1730 of the fingerprint sensor package 1700 may be greater than a height of a region in which the holes 1731 are formed, and thus an edge region of the molding 1730 of the fingerprint sensor package 1700 may be attached to the display panel.

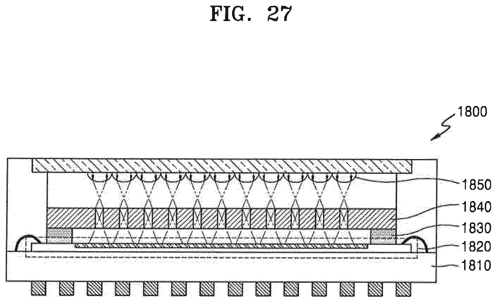

FIG. 27 is a diagram illustrating a structure of a fingerprint sensor package according to another example embodiment of inventive concepts.

Referring to FIG. 27, a fingerprint sensor package 1800 may include a package substrate 1810, an image sensor 1820 mounted on the package substrate 1810, and a pinhole mask 1840 located on the image sensor 1820. Also, a support 1830 for supporting the pinhole mask 1840 may be formed on the image sensor 1820. In the same or similar way as described above, the image sensor 1820 may include a sensor chip and a logic chip, and a molding corresponding to a package cover may be formed in the fingerprint sensor package 1800. Also, the fingerprint sensor package 1800 may be attached to a display panel including a light source such as an OLED or the like.

According to one example embodiment, the fingerprint sensor package 1800 may further include a light-intensity boosting micro lens array 1850. The micro-lens array 1850 may be located on an upper portion of the fingerprint sensor package 1800, and may include a plurality of micro-lenses corresponding to a plurality of pinholes included in the pinhole mask 1840. For example, the plurality of micro-lenses included in the micro-lens array 1850 may be aligned with the pinholes. Also, a molding located at an edge region in the fingerprint sensor package 1800 may be used as a unit for supporting the micro-lens array 1850. For example, the micro-lens array 1850 may be attached to the molding located at the edge region.

According to the structure illustrated in FIG. 27, light emitted from a light source (e.g., an OLED) of the display panel may be reflected by a fingerprint and transmitted to the image sensor 1820 through the micro-lens array 1850 and the pinhole mask 1840. In this case, in a path of the light transmitted to the image sensor 1820, a focus by the micro-lens array 1850 may be formed, and a focus by the pinhole mask 1840 may be formed. That is, by such a structure, the light collected by the micro-lens array 1850 may be transmitted to the pinhole mask 1840, and an amount of light sensed by the image sensor 1820 may be increased.

Also, since two focuses are formed in the light propagation path to the image sensor 1820, an image reversal which occurs when passing through the focus occurs twice, and thus the image sensed by the image sensor 1820 may correspond to an original fingerprint image. Also, when a distance between the fingerprint pixel and the pinhole mask 1840 is relatively large, a ratio of a diameter of the pinhole to a thickness thereof is relatively small. However, according to an example embodiment, since a concentrating effect is generated by the micro-lens array 1850 and the pinhole mask 1840 passes light from a closely located micro-lens of the micro-lens array 1850, an angle of view of the pinhole may be increased. That is, according to the embodiment, since a process condition for implementing the ratio of the thickness of the pinhole to the diameter thereof may be loosened, a thickness of the pinhole mask 1840 may be reduced. Therefore, a manufacturing process of the pinhole mask 1840 may be made easier.

Meanwhile, when the pinhole mask 1840 is implemented with an opaque element, the pinhole mask 1840 may also function to block other lights (i.e., lights unrelated to fingerprint sensing) transmitted through a region except for the micro-lens array 1850.

Also, according to one example embodiment, the micro-lenses of the micro-lens array 1850 may be located to be aligned with the sensor pixels of the image sensor 1820 together with the pinholes of the pinhole mask 1840. According to one example embodiment, when the micro-lenses of the micro-lens array 1850 are not aligned with the sensor pixels, the image sensor 1820 may perform a compensation operation on offsets between the micro-lenses and the sensor pixels in software. For example, the sensor pixel may include a plurality of PDs (or a plurality of sub-pixels), a center of the pinhole may be determined by sensing light transmitted to the sensor pixel, the offset compensation operation may be performed in software, and thus an electrical signal (or corresponding image data) corresponding to a case in which the sensor pixel is aligned with the center of the pinhole may be generated.

Although an example in which the pinhole mask 1840 is attached to the image sensor 1820 in the packaging process is illustrated as an externally mounted method in the above-described embodiment, the embodiment may be variously modified. For example, in the above-described embodiment using the micro-lens array 1850, the pinhole mask 1840 may be embedded in the image sensor 1820 in a process for implementing the image sensor 1820. According to the above-described embodiment, a sustain wafer having a plurality of holes may perform a function of the pinhole mask 1840 as an embedded method.

Also, the fingerprint sensor packages 1700 and 1800 of FIGS. 26 and 27 may be mounted on the boards in a COB form according to the above-described embodiments. Alternatively, the fingerprint sensor packages 1700 and 1800 may be mounted on films, in which wirings are embedded and holes having any shape (e.g., a large rectangular shape) are formed so that light may be transmitted through a surface of a sensor, in a COF form.

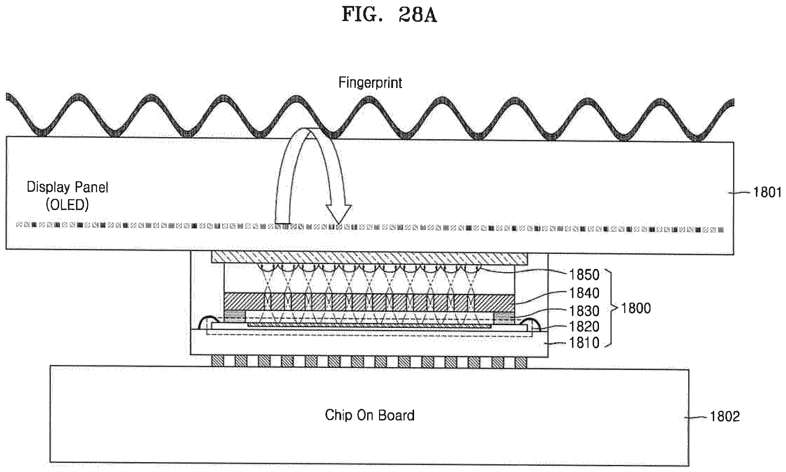

FIGS. 28A to 28C are diagrams illustrating a fingerprint sensing system including a fingerprint sensor package to which a micro-lens array is applied.

Referring to FIG. 28A, the fingerprint sensing system may include the fingerprint sensor package 1800 in the above-described embodiment. Also, the fingerprint sensing system may include a display panel 1801 to which the fingerprint sensor package 1800 is attached and a board 1802 for mounting the fingerprint sensor package 1800. Since the micro-lens array 1850 is located at an upper portion of the fingerprint sensor package 1800, the micro-lens array 1850 may be attached to one surface of the display panel 1801. Although not illustrated in FIG. 28A, the micro-lens array 1850 may be attached to one surface of the display panel 1801 through an adhesive element having a plane form or a window frame form.

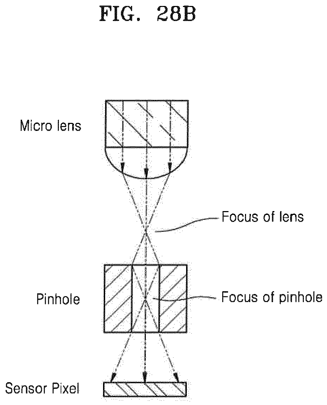

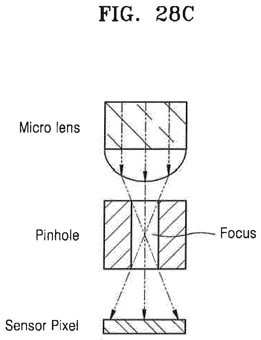

Meanwhile, FIGS. 28B and 28C illustrate light propagation paths in the fingerprint sensor package 1800 of the fingerprint sensing system. In FIG. 28B, an example in which positions of micro-lenses, pinholes, and sensor pixels are aligned is illustrated. As illustrated in FIG. 28B, light emitted from an OLED as a light source of the display panel 1801 is reflected by a fingerprint of the user, transmitted in a direction towards a backplane, and provided to the micro-lens array 1850. Also, as the micro-lens array 1850 and the pinhole mask 1840 are disposed in the fingerprint sensor package 1800, a focus of the light formed by the micro-lens array 1850 and a focus of the light formed by the pinhole mask 1840 are present. Also, a diameter and height of the pinhole may be determined in consideration of a relatively short distance from the micro-lens array 1850 regardless of a distance between the pinhole and the fingerprint of the user. In the present embodiment, since the ratio of the thickness of the pinhole to the diameter thereof does not need to be relatively large, the implementation of the pinhole mask 1840 is facilitated. Alternatively, as illustrated in FIG. 28C, the pinhole may be disposed to be located at a focal height that the micro-lens makes. In this case, the overall height from the micro-lens to the sensor pixel may be reduced compared to that in the embodiment of FIG. 28B.

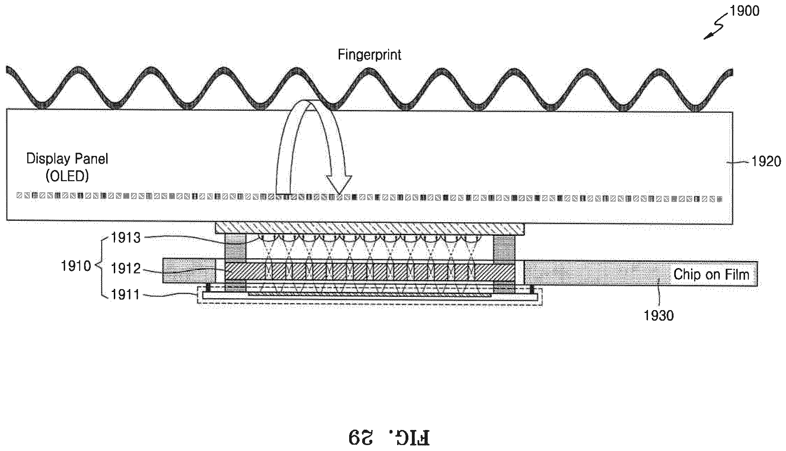

FIG. 29 is a diagram illustrating an example in which a fingerprint sensor package, to which a micro-lens array is applied, is mounted on a film in which a wiring is embedded.

Referring to FIG. 29, a fingerprint sensing system 1900 may include a fingerprint sensor package 1910, a display panel 1920, and a film 1930 for mounting the fingerprint sensor package 1910. The fingerprint sensor package 1910 may include an image sensor 1911, a pinhole mask 1912, and a micro-lens array 1913. Also, a hole having a size so that the fingerprint sensor package 1910 may pass therethrough may be formed in the film 1930 on which the fingerprint sensor package 1910 is mounted, and wirings for electrical transmission of signals may be formed on at least one surface of the film 1930.

According to one example embodiment, the fingerprint sensor package 1910 may be attached to the display panel 1920 through the hole of the film 1930. Also, although not illustrated in FIG. 29, an adhesive element in a plane form or window frame form for attaching the fingerprint sensor package 1910 to the display panel 1920 may be further provided in the fingerprint sensing system 1900. Also, the image sensor 1911 may include a light-receiving region and a logic region, and the image sensor 1911 may include a sensor chip including the light-receiving region and a logic chip including the logic region when the light-receiving region and the logic region are implemented as separate chips. The image sensor 1911 may be electrically connected to one surface of the film 1930 through bonding.

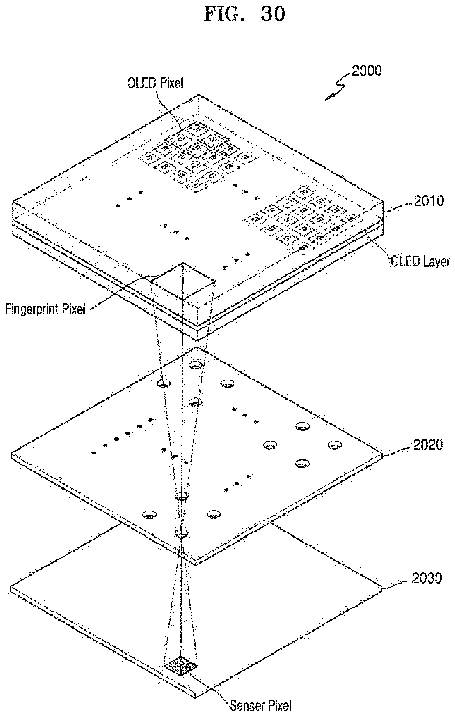

FIGS. 30 to 32 are diagrams illustrating fingerprint sensing systems according to example embodiments of inventive concepts.

Referring to FIG. 30, a fingerprint sensing system 2000 may include a display panel 2010 including a plurality of OLEDs, a pinhole mask 2020 in which a plurality of pinholes are formed, and an image sensor 2030 including a plurality of sensor pixels. Each of the sensor pixels may include one or more PDs. Also, the plurality of OLEDs may be located in an OLED layer of the display panel 2010.

The plurality of OLEDs of the display panel 2010 may be elements which emit light having different colors and the wavelength bands by themselves. For example, the display panel 2010 may include an OLED which emits red (R) color, an OLED which emits blue (B) color, and an OLED which emits green (G) color. When the OLEDs emit light having different colors, wavelengths of light of respective colors may have different values.

According to one example embodiment, OLEDs which emit any one color among the OLEDs which emit a plurality of colors may be selectively used in relation to a fingerprint sensing operation. For example, light from the OLED which emits the red (R) color may be reflected by a fingerprint and provided to the image sensor 2030 through the pinhole mask 2020. In this case, when only the OLED which emits the red (R) color is selectively activated in relation to the fingerprint sensor, a separate color filter needs not to be disposed in the image sensor 2030. Alternatively, only a red color filter corresponding to an OLED in which only the red (R) color is selectively activated to emit light may be implemented in the image sensor 2030 as a mono filter for filtering only one color corresponding to a plurality of PDs. That is, a color filter through which only the same color passes may be used according to the selected color of the light source, or a filter may not be disposed in the image sensor 2030.

When using OLEDs which emit a plurality of colors, a difference in a focus length due to chromatic aberration may occur for each wavelength of light. In this case, since the focuses may not be concentrated into one, the sharpness in a sensing result may be degraded. On the other hand, according to the above-described embodiment, since a fingerprint sensing operation according to example embodiments of inventive concepts is performed selectively using only an OLED, which emits light having the same wavelength, among the plurality of OLEDs, a uniform focus may be formed in a plurality of pinholes in one pinhole mask, and thus the sharpness of the fingerprint sensing result may be improved.

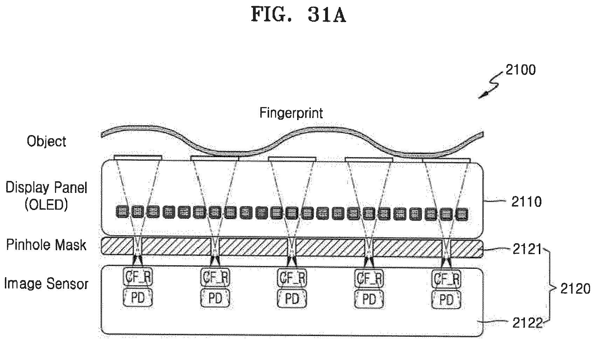

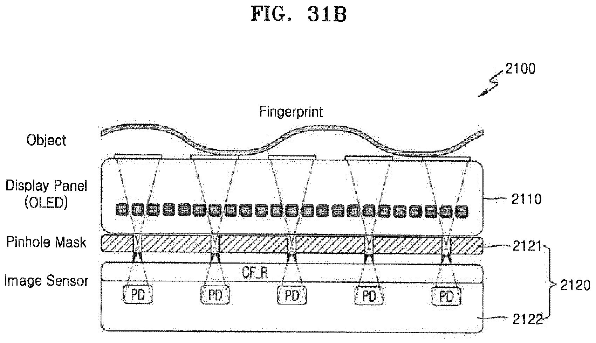

Meanwhile, FIGS. 31A and 31B illustrate another example in which sensing sharpness is improved as in the above-described embodiment.

Referring to FIG. 31A, a fingerprint sensing system 2100 may include a display panel 2110 and a fingerprint sensor 2120 and the fingerprint sensor 2120 may include a pinhole mask 2121 and an image sensor 2122. According to the above-described embodiment, the fingerprint sensor 2120 may be packaged and attached to one surface of the display panel 2110.

The pinhole mask 2121 may include a plurality of pinholes and each of the pinholes may form a focus of light reflected and transmitted by a fingerprint. Also, the display panel 2110 may include OLEDs which emit light of a plurality of colors by themselves without backlight, and OLEDs which emit light of at least some of the plurality of colors among the OLEDs may be used for a fingerprint sensing operation.

The image sensor 2122 may include a plurality of sensor pixels corresponding to a plurality of pinholes, and each of the sensor pixels may include one or more PDs. Also, according to one example embodiment, a color filter may be formed to correspond to each of the plurality of sensor pixels, and the same color filter (or a filter through which light having the same wavelength passes) may be formed to correspond to the sensor pixels. In the embodiment of FIG. 31A, an example in which red color filters CF_R are formed on top of PDs in the image sensor to correspond to the sensor pixels is illustrated.

According to the embodiment illustrated in FIG. 31A, since only light of the same color (or the same wavelength) may be selectively provided to the PDs in the sensor pixels through a mono filter for filtering the same color in the image sensor 2122 even when light from all the OLEDs of the display panel 2110 is reflected by the fingerprint, the sharpness of the fingerprint sensing result may be improved similarly in the above-described embodiment.

Meanwhile, in FIG. 31B, an example in which a process in which a color filter is formed in a front surface of the sensor region as well as on a top of a PD is used according to one example embodiment of inventive concepts is illustrated.

Meanwhile, although an example in which a pinhole mask is provided in an externally mounted form is illustrated in the above-described embodiments, a pinhole mask having an embedded structure in the above-described embodiment may be provided. For example, in the embodiments of FIGS. 31A and 31B, when a sustain wafer is stacked on the PDs in the image sensor 2122 process to perform a pinhole mask function, the pinhole may be located between the red color filter CF_R and the PDs.

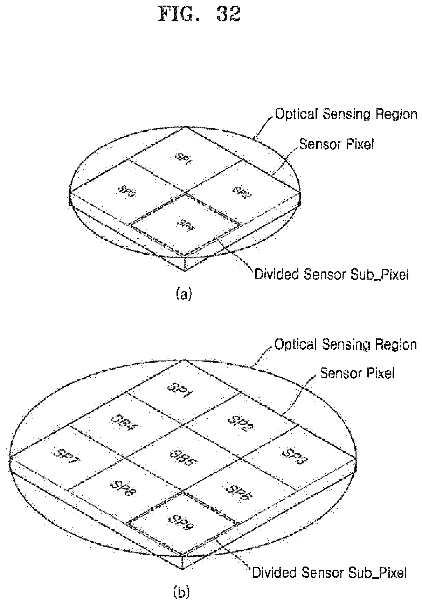

Meanwhile, in FIGS. 32A and 32B, an example in which a spatial resolution in the fingerprint sensor is increased is illustrated.

Referring to FIGS. 32A and 32B, a sensor pixel corresponding to one pinhole may be divided into a plurality of sub-pixels. An example in which one sensor pixel is divided into four sub-pixels SP1 to SP4 is illustrated in FIG. 32A, and an example in which one sensor pixel is divided into nine sub-pixels SP1 to SP9 is illustrated in FIG. 32B. Also, portions represented as circles in the sensor pixels of FIGS. 32A and 32B may respectively correspond to optical sensing regions of the sensor pixels. Also, each of the sub-pixels may generate an electrical signal according to a result of sensing light.

In order to additionally obtain a resolution of the fingerprint image, a pitch of the fingerprint pixel is reduced. To this end, an angle of view of each pinhole is reduced by reducing a diameter thereof, and a pitch between the pinholes is reduced. However, increasing the resolution by reducing the diameter or pitch of the pinhole may cause limitations in a manufacturing process.

According to the example embodiment of inventive concepts illustrated in FIGS. 32A and 32B, the resolution of the fingerprint sensing result may be increased, and thus a fingerprint image having a high resolution may be obtained. For example, when 2^{N} sensor pixels corresponding to 2^{N} pinholes are disposed in the image sensor and each of the sensor pixels is divided into 2^{M} sub-pixels, electrical signals may be generated according to sensing results from all 2^{N+M} sub-pixels. That is, since the resolution of the entire image data corresponding to the sensing results corresponds to N+M bits even when 2^{N} pinholes are disposed, the resolution thereof may be increased by M bits without increasing the number of pinholes.

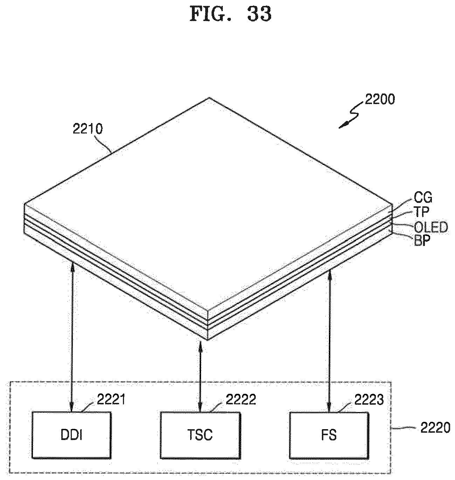

FIG. 33 is a block diagram illustrating a processing system including the fingerprint sensors or fingerprint sensor packages according to example embodiments of inventive concepts.

Referring to FIG. 33, a fingerprint sensing system 2200 may include a display panel 2210 and a processing system 2220. The display panel 2210 may include a cover glass CG, a touch panel TP including a plurality of sensing units, a display region including a plurality of OLEDs, and a backplane BP. The touch panel TP may be disposed to sense a touch operation of the user. For example, when a touch panel TP using a capacitance method is applied, the touch panel TP may include sensing units of which capacitance values vary based on a user's touch.

The processing system 2220 may include a display driving circuit 2221, a touch screen controller 2222, and a fingerprint sensor 2223. The display driving circuit 2221 may perform various control operations for implementing an image on the display panel 2210. For example, the display driving circuit 2221 may provide gradation data related to image implementation on the display panel 2210. Also, the touch screen controller 2222 may sense a capacitance change of the sensing units of the touch panel TP to generate a touch sensing result. For example, the touch screen controller 2222 may provide a driving signal for driving the touch panel TP, and receive and process an electrical signal corresponding to the capacitance change of the sensing units in the touch panel TP. Also, the fingerprint sensor 2223 may correspond to any of the various embodiments described above, and generate a fingerprint image by using OLEDs as light sources and sensing light reflected by a fingerprint of the user.

Meanwhile, according to one example embodiment, the processing system 2220 may be implemented as one semiconductor chip. For example, functions of the display driving circuit 2221, the touch screen controller 2222, and the fingerprint sensor 2223 may be integrated on one semiconductor substrate. According to one example embodiment, the fingerprint sensor 2223 may be implemented to include at least some of various functions described in the above-described embodiments. For example, as the fingerprint sensor 2223 includes a PD, the fingerprint sensor 2223 may include a light-receiving region, or may further include a logic region for generating the image data described in the above-described embodiment.

According to one example embodiment, the display driving circuit 2221, the touch screen controller 2222, and the fingerprint sensor 2223 may be formed in the same semiconductor chip to transmit and receive various pieces of information or signals. For example, an operation of the fingerprint sensor 2223 may be controlled by the display driving circuit 2221 and/or the touch screen controller 2222. For example, power and control signals required for sensing timing or a sensing operation of the fingerprint sensor 2223 may be generated by the display driving circuit 2221 and/or the touch screen controller 2222.

In the embodiment illustrated in FIG. 33, an example in which the display driving circuit 2221, the touch screen controller 2222, and the fingerprint sensor 2223 are integrated into a single semiconductor chip is illustrated, but inventive concepts are not limited thereto. In example embodiments of inventive concepts, the fingerprint sensor 2223 may be implemented in the same semiconductor chip as the display driving circuit 2221, or the fingerprint sensor 2223 may be implemented in the same semiconductor chip as the touch screen controller 2222 or may be implemented as a separate semiconductor chip.

Meanwhile, although some example embodiments applied to inventive concepts have been described with reference to the separate drawings, the fingerprint sensor, the fingerprint sensor package, or the fingerprint sensing system according to example embodiments of inventive concepts may be configured by combining two or more embodiments.

The technical scope of inventive concepts is for a fingerprint recognition field which is most widely used among biometrics fields in which market demand is increasing, and provides a structure of an on-display fingerprint sensor for a smart phone or a wearable device. For example, currently, in most smart phones, a fingerprint sensor is mounted on a home button which is located at a lower center of the phone, or a back surface of the phone. According to example embodiments of inventive concepts, an optical sensor structure for directly recognizing a fingerprint on a display of the smart phone without the fingerprint sensor mounted on a home button, which is located at a lower center of the phone or a back surface of the phone may be provided.

In the optical fingerprint sensor, the fingerprint sensor package, and the fingerprint sensing system according to the technological scope of inventive concepts, since a thickness of the fingerprint sensor package can be minimized, an ultra-thin fingerprint sensing system can be provided.

Also, in the optical fingerprint sensor, the fingerprint sensor package, and the fingerprint sensing system according to the technological scope of inventive concepts, since light sources for a display operation which is provided in a display panel are used as light sources for optical sensing of a fingerprint, there is no need to add additional light sources, so that a manufacturing cost can be reduced.

While inventive concepts have been particularly shown and described with reference to embodiments thereof, it will be understood that various changes in form and details may be made therein without departing from the spirit and scope of the following claims.

## Claims

1. A fingerprint sensor comprising:
an image sensor including a plurality of sensor pixels, the sensor pixels configured to sense light reflected by a fingerprint and generate image information corresponding to the fingerprint; and
a pinhole mask defining a plurality of pinholes, each of the pinholes forming a focus for transmitting the light reflected by the fingerprint to the image sensor, and the light is emitted from a plurality of organic light-emitting diodes (OLEDs) and is reflected by the fingerprint.

2. The fingerprint sensor of claim 1, wherein the image sensor includes:
photodiodes (PDs) in the plurality of sensor pixels, the PDs configured to output an electrical signal having the image information; and
a logic circuit configured to generate image data by signal processing the electrical signal, generate a fingerprint image using the generated image data, and output the fingerprint image, the logic circuit being located separate from the PDs.

3. The fingerprint sensor of claim 1 or 2, wherein the fingerprint sensor is configured to sense the light which is emitted from the plurality of OLEDs in a direction towards a cover glass and reflected by the fingerprint from the cover glass in a direction towards a backplane.

4. The fingerprint sensor of claim 3, wherein the plurality of OLEDs have a plurality of colors.

5. The fingerprint sensor of claim 3 or 4, wherein the pinhole mask is between an OLED display panel and the image sensor, the OLED display panel including the plurality of OLEDs.

6. The fingerprint sensor of any one of claims 3 to 5, wherein the image sensor includes a color filter through which light having a same color as the color filter selectively passes to a corresponding portion of the plurality of sensor pixels to selectively sense the light having the same color and a same wavelength band among the plurality of OLEDs.

7. The fingerprint sensor of any one of claims 1 to 6, wherein:
the plurality of sensor pixels correspond to the plurality of pinholes, respectively; and
one photodiode (PD) is in each of the plurality of sensor pixels.

8. The fingerprint sensor of any one of claims 1 to 6, wherein:
the plurality of sensor pixels correspond to the plurality of pinholes, respectively; and
a plurality of photodiodes (PDs) are in each of the plurality of sensor pixels.

9. The fingerprint sensor of any one of claims 1 to 8, wherein:
the pinhole mask is in the image sensor;
the image sensor includes a semiconductor substrate and one or more metal layers on the semiconductor substrate, the semiconductor substrate including a plurality of photodiodes (PDs); and
the pinhole mask includes one or more metal layers and the one or more metal layers define the plurality of pinholes, the plurality of pinholes are in a region in which a metal line is not in the one or more metal layers.

10. The fingerprint sensor of any one of any one of claims 1 to 9, wherein:
the pinhole mask is in the image sensor;
the image sensor includes a semiconductor substrate and one or more metal layers, the semiconductor substrate including a plurality of photodiodes (PDs); and
the pinhole mask is on the semiconductor substrate or the one or more metal layers.

11. The fingerprint sensor of any one of claims 1 to 9, wherein the fingerprint sensor is a semiconductor package, the pinhole mask is on a semiconductor chip, the semiconductor chip includes the image sensor, and the fingerprint sensor further includes:
a support on the semiconductor chip, the support supporting the pinhole mask.

12. A fingerprint sensing system comprising:
a display panel including light sources, the light sources configured to emit light having one or more colors in relation to a display operation;
an image sensor attached to one surface of the display panel and configured to sense light from the light sources that is reflected by a fingerprint located on another surface of the display panel, the image sensor configured to generate image information according to a sensing result; and
a pinhole mask between the display panel and the image sensor, the pinhole mask having a plurality of pinholes for forming a focus of the light reflected by the fingerprint,
wherein, in a fingerprint sensing operation, the image sensor senses light having some of the one or more colors and generates the image information.

13. The fingerprint sensing system of claim 12, wherein, in the fingerprint sensing operation, the display panel selectively activates one of the light sources.

14. The fingerprint sensing system of claim 12, wherein:
in the fingerprint sensing operation, the display panel activates a portion of the light sources, the portion of the light sources emit light having at least one of the one or more colors; and
the image sensor includes a color filter for selectively sensing light having any one of the at least one color.

15. The fingerprint sensing system of any one of claims 12 to 14, wherein the display panel is an organic light-emitting diode (OLED) display panel including a plurality of OLEDs as the light sources.

16. A sensing system comprising:
a display panel configured to generate light and receive reflected light based on the generated light; and
a sensor, the sensor including,
a mask defining a plurality of holes, the mask configured to guide a first portion of the reflected light through the mask, a first side of the mask being on the display panel, and
an image sensor on a second side of the mask, the image sensor including a plurality of sensor pixels, the plurality of sensor pixels configured to sense the first portion of the reflected light and generate electrical signals based on the sensed light, the mask being configured to guide the first portion of the reflected light to the image sensor without a lens.

17. The sensing system of claim 16, wherein the mask includes,
a first layer including first metal wirings, and
a second layer including second metal wirings, the first metal wirings being perpendicular to the second metal wirings, the first metal wirings and the second metal wirings configured to block a second portion of the reflected light.

18. The sensing system of claim 17, wherein the first layer and the second layer define the holes through the mask.

19. The sensing system of claim any one of claims 16 to 18, wherein the display panel includes a plurality of organic light-emitting diodes (OLEDs) configured to generate the generated light.

20. The sensing system of any one of claims 16 to 19, wherein the mask is adjacent to the image sensor.
